# EUROPEAN PATENT SPECIFICATION

(11) **EP 1 864 995 B1**
(45) Date of publication and mention of the grant of the patent: **08.12.2010**
(21) Application number: 04791400.7
(22) Date of filing: 30.10.2004
(51) Int. Cl.: C07K 7/64

(54) **BETA-LACTAMIC RGD CYCLOPEPTIDES CONTAINING GAMMA TURNS**
B-LACTAM-RGD-CYCLOPEPTIDE MIT GAMMA-(G-)WENDUNGEN
CYCLOPEPTIDES RGD BETA-LACTAMIQUES CONTENANT DES TOURS GAMMA

(43) Date of publication of application: 12.12.2007
(73) Proprietor: UNIVERSIDAD DEL PAIS VASCO-EUSKAL HERRIKO UNIBERSITATEA, 48940 Leioa (ES)
(72) Inventor: AIZPURUA IPARRAGUIRRE, Jesús Ma, E-20018 San Sebastian (ES); GAMBOA LANDA, José Ignacio, E-20018 San Sebastian (ES); PALOMO NICOLAO, Claudio, E-20018 San Sebastian (ES); LOINAZ BORDONABE, Iradia, E-20018 San Sebastian (ES); MIRANDA MURUA, José Ignacio, E-20018 San Sebastian (ES)
(74) Representative: Isern-Jara, Nuria
(86) International application number: PCT/ES2004/000481
(87) International publication number: WO 2006/048473

(56) References cited:
- EP-A2- 1 077 218
- WO-A2-2004/005328
- US-A- 5 693 612
- DATABASE CAS [Online] BELVISI L. ET AL.: 'Cyclic RGD peptides containing azabicycloalkane reverse-turn mimics', XP003018414 Retrieved from STN Database accession no. (139:53275) & HELVETICA CHIMICA ACTA vol. 85, no. 12, 2002, pages 4353 - 4368
- DATABASE CAS [Online] BELVISI L. ET AL.: 'Conformational analysis of azabicycloalkane amino acid scaffolds as reverse-turn inducer dipeptide mimics', XP003018415 Retrieved from STN Database accession no. (133:267124) & EUROPEAN JOURNAL OF ORGANIC CHEMISTRY vol. 14, 2002, pages 2563 - 2569
- DATABASE CAS [Online] PALOMO C. ET AL.: 'Development of a new family of conformationally restricted peptides as potent nucleators of beta-turns. Design synthesis, structure and biological evaluation of a beta-Lactam peptide analogue of melanostatin', XP003018416 Retrieved from STN Database accession no. (140:146498) & JOURNAL OF AMERICAN CHEMICAL SOCIETY vol. 125, no. 52, 2003, pages 16243 - 16260
- BELVISI L. ET AL.: 'Potent integrin antagonists from a small library of RGD-including cyclic pseudopeptides' ORGANIC LETTERS vol. 3, no. 7, 2001, pages 1001 - 1004, XP001037188

## Description

### FIELD OF THE INVENTION

The present invention relates to new RGD β-lactam cyclopeptide compounds and their pharmaceutically acceptable salts, the procedures for their preparation and the pharmaceutical compositions derived thereof, as well as their use to prepare drugs for human and animal therapy. It also relates to the materials resulting from their immobilization on proteins, polymers, stationary phases for chromatography or fluorescent dyes and the use of the resulting materials to manufacture systems for analysis or diagnostic, separation systems or chromatographic stationary phases for integrin detection.

### BACKGROUND ART

Cell adhesion and interaction properties within different cell types and with the components of the extracellular matrix are essential for cell function. Integrins are αβ dimeric glycoprotein receptors of the cell wall playing an important role in such processes.

For instance, αvβ3 integrin is an RGD-dependent receptor of endothelial cells preferentially expressed in angiogenic veins. It has been shown to play an important role during neovascularization and its antagonists block the formation of new blood vessels without affecting the pre-existing ones (Brooks PC et al. Science 1994 264(5158): 569-571 "Requirement of vascular integrin αvβ3 for angiogenesis*").*

Several integrins recognize the hydrophilic amino acid sequence RGD Arg-Gly-Asp (Ruoslahti E, Pierschbacher M, Cell 1986 44:517-518 " Arg-Gly-Asp: A versatile cell recognition signal*"*)*.* This sequence occurs in adhesive proteins such as fibronectin, vitronectin, fibrinogen, von Willebrand factor and collagen. Platelet aggregation, tumoral cell migration, metastasis, angiogenesis and osteoclast adhesion to the bone matrix depend on the interaction between integrin-type receptors and the proteins of the extracellular matrix mentioned above (Tissue Res. 305, 285-295 (2001).

Thus, antibodies, peptides or peptidomimetics blocking selectively cell adhesion mechanisms controlled by one or several integrins and, more particularly by αvβ3 integrin, are antiangiogenic agents with application in antitumoral therapy, as shown for instance in Science, 264, 569-571 (1994), Cell, 79, 1157-1164 (1994), Science, 270, 1500-1502 (1995) or J. Med. Chem., 43, 22-26 (2000). They are also useful for the treatment of restenosis following percutaneous transluminal coronary angioplasty (PTCA), of rheumatoid arthritis or osteoporosis.

Synthetic peptides containing the RGD sequence might compete favorably with proteins in binding receptors (integrins). It has been shown that conformation of the RGD sequence is crucial for specific binding to the receptor. The presence of γ and/or β turned conformations blocked in cyclopeptides or equivalent pseudopeptides can be determined in solution by NMR techniques, as described for instance in Quart. Rev. Biophys. 31, 142-237 (1998), and methods for the conformational blocking of different combinations of γ and/or β turns have been used to enhance the potency and selectivity of RGD pseudocyclopeptides recognizing integrins of the αᵥβ₃, α_{II}β₃, αᵥβ₅ and α₅β₁ families, as shown for instance in J. Biol. Chem. 269, 20233-20238 (1994).

It is well known that certain RGD cyclopentapeptide mimetics possessing a γ turn and a β turn within the Arg-Gly-Asp residues inhibit moderately the αᵥβ₃ integrin. Cyclopentapeptides, α-amino-γ-lactams, α-amino-δ-lactams, α-amino-ε-lactams, prolines, spirolactam prolines and bicyclic lactams have been used to stabilize simultaneously a γ turn and a β turn within the Arg-Gly-Asp residues, as described for example in J. Am. Chem. Soc. 118, 7881-7891 (1996), Org. Lett. 3, 1001-1004 (2001) or EP-683 173. The presence of γ and β turns in such compounds is characterized by the formation of hydrogen bonds; the former between NH(Asp) and C=O(Arg), corresponds to a γ turn and the second between NH(Arg) and C=O(Asp), corresponds to β turn. Simultaneous stabilization of the same kind of γ and β turns within the Arg-Gly-Asp residues in RGD cyclopentapeptides without the incorporation of lactams or other cyclic agents is also known. It has been achieved, for instance, by changing the (D) and (L) configuration of some amino acids of the cycle, see EP-0 606 881.

On the other hand, it is known that RGD cyclopentapeptide cyclo(Arg-Gly-Asp-(D)Phe-N(Me)Val), containing simultaneously a D-amino acid and an N-alkyl residue, presents only weak γ-turns in water solution, as established in J. Med. Chem. 42, 3033-3040 (1999). This compound, having no β turn in aqueous solution, displays a higher affinity for αᵥβ₃ integrin than its analogues containing β and γ turns. Hence, modification of RGD cyclopeptides or other structural elements to stabilize a γ turn, preferably without forming β turns, seems a desirable requirement to enhance the inhibitory activity of such compounds against αᵥβ₃ integrin.

In view of the state of the art, it still persists the need for selective and highly active integrin inhibitors. Particularly suitable are cyclic RGD compounds incorporating stable γ turns displaying good pharmacological properties.

### SUMMARY OF THE INVENTION

It has been found that cyclic pseudopeptides containing the RGD sequence and an alpha-amino beta-lactam residue present a γ turn conformation and have good integrin inhibition properties. The compounds of the invention are characterized because they are conformationally restricted, regardless of their substituents, and because they contain at least one γ turn stable in water solution.

One aspect of the invention includes the compounds of the formula (I):

*cyclo*[Arg-Gly-Asp-(beta-Lactam)] (I)

containing at least one γ turn, where (beta-Lactam)- is a fragment of 3-amino-1-(2-carbonylmethyl)-azetidin-2-one of the formula (II) 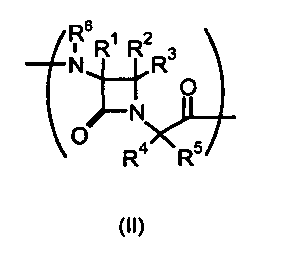 where R¹, R², R³, R⁴ , R⁵ y R⁶ are independently selected from the group formed by hydrogen, substituted or unsubstituted alkyl, substituted or unsubstituted cycloalkyl, substituted or unsubstituted alkenyl, substituted or unsubstituted aryl, substituted or unsubstituted heterocyclyl, - COR⁷, -C(O)OR⁷, -C(O)NR⁷R⁸ -C=NR⁷, -CN, -OR⁷,-OC(O)R⁷, -S(Oh-R⁷ , -NR⁷R⁸, -NR⁷C(O)R⁸, - NO₂, -N=CR⁷R⁸ or halogen;
where two of these groups can be linked to each other forming a cyclic substituent;
t is 1, 2 or 3,
R⁷ and R⁸ are selected independently from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxide, substituted or unsubstituted aryloxyl, halogen;
their enantiomers, diastereomers or a pharmaceutically acceptable salt.

Another aspect of the invention includes pharmaceutical compositions comprising a compound defined as above, its enantiomers, diastereomers or their mixtures.

The invention also includes a procedure to obtain the compounds defined above, comprising:
a) The cyclization of the following pseudopeptides protected at the Arg and Asp residues:

   H-Arg-Gly-Asp-(beta-Lactam)-OH

   H-Gly-Asp-(beta-Lactam)-Arg-OH

   H-Asp-(beta-LactamrArg-Gly-OH

   H-(beta-Lactam)-Arg-Gly-Asp-OH

   Where (beta-Lactam)- is a fragment of 3-amino-1-(2-carbonylmethyl)-azetidin-2-one of the formula (II) as defined above;
b) optionally, the elimination of the protecting groups.

The invention also includes the intermediate compounds necessary for the procedure containing the structural elements defined above and, more particularly, the pentapeptides of the formulae

H-Arg-Gly-Asp-(beta-Lactam)-OH

H-Gly-Asp-(beta-Lactam)-Arg-OH

H-Asp-(beta-Lactam)-Arg-Gly-OH

H-(beta-Lactam)-Arg-Gly-Asp-OH

where (beta-Lactam)- is a fragment of 3-amino-1-(2-carbonylmethyl)-azetidin-2 -one of the formula (II) defined as above, with the Asp and Arg residues optionally protected with protecting groups.

Another aspect of the invention refers to the use of compounds defined as above for drug manufacturing. Preferably, for the treatment of diseases mediated by the over-expression of integrins αᵥβ₃, α_{II}β₃, αᵥβ₅ and α₅β₁ in humans or animals, including cancer, metastasis of cancerous solid tumors, thrombosis, restenosis following percutaneous transluminal coronary angioplasty (PTCA), rheumatoid arthritis or osteoporosis.

An additional aspect of the invention refers to systems for the purification of integrins and diagnostic or analysis systems comprising a pseudopeptide defined as above.

Finally, the compounds of the invention can be immobilized on supports to form materials, which are also included in the invention. Supports comprise, polymers, proteins, fluorescent dyes and stationary chromatographic phases, among others.

### SHORT DESCRIPTION OF THE CONTENTS OF FIGURES:

**Figure 1** shows the ¹H-NMR (500MHz) (WATERGATE) spectrum of Compound A (10mM) recorded in H₂O/D₂O 90/10 solvent at 300K
**Figure 2** shows the TOCSY(500MHz) (WATERGATE) spectrum of Compound A (10mM) recorded in H₂O/D₂O 90/10 solvent at 300K
**Figure 3** shows the ROESY spectrum of Compound A (10mM) recorded in H₂O/D₂O 90/10 solvent at 300K with a mixing time of 200ms.
**Figure 4** shows (top) thermal shift of amide protons of Compound A (10mM) recorded in H₂O/D₂O 90/10 solvent between 300K and 325K at 5K intervals and (bottom) the thermal coefficients (ppb/K) of the amide protons of Compound A.
**Figure 5** shows the tridimensional structure of Compound A in H₂O/D₂O 90/10 solution at 300K determined by Molecular Dynamics, including inter-proton distance constraints obtained from NMR (ROESY) experiment.
**Figure 6** shows the ¹H-NMR (500MHz) (WATERGATE) spectrum of Compound B (5mM) recorded in H₂O/D₂O 90/10 solvent at 300K.
**Figure 7** shows the TOCSY(500MHz) (WATERGATE) spectrum of Compound B (5mM) recorded in H₂O/D₂O 90/10 solvent at 300K.
**Figure 8** shows the ROESY spectrum of Compound B (5mM) measured in H₂O/D₂O 90/10 solvent at 300K with a mixing time of 400ms.
**Figure 9** shows (top) thermal shift of amide protons of Compound B (5mM) recorded in H₂O/D₂O 90/10 solvent between 300K and 325K at 5K intervals and (bottom) the thermal coefficients (ppb/K) of the amide protons of Compound B.
**Figure 10** shows the tridimensional structure of Compound B in H₂O/D₂O 90/10 solution at 300K determined by Molecular Dynamics, including inter-proton distance constraints obtained from NMR (ROESY) experiment.
**Figure 11** shows the curves of the inhibition of the adhesion on a vitronectin surface of αᵥβ₃ integrin over-expressed human umbilical vein endothelial cells (HUVEC) by the addition of Compounds A, and B. Each plotted curve represents the mean value of three determinations and error bars indicate standard deviation for the measurement of each value.

### DETAILED DESCRIPTION OF THE INVENTION

In the present description, the indicated terms are defined as follows:
"Alkyl" refers to a hydrocarbon chain radical, linear or branched, constituted of carbon and hydrogen atoms, without unsaturation, containing one to twelve carbon atoms, and linked to the main molecule by a single bond; for instance, methyl, ethyl, n-propyl, iso-propyl, n-butyl, n-pentyl, etc. Alkyl radicals may optionally contain one or more substituents such as aryl, halogen, hydroxy, alkoxy, carboxy, cyano, carbonyl, acyl, alkoxycarbonyl, amino, nitro, mercapto, alkylthio, etc. Upon substitution by an aryl group, an "Aralkyl" radical is obtained, such as benzyl or phenethyl.
"Aryl" refers to single- and multiple ring radicals, including multiple rings containing separated and/or condensed rings. Typical aryl groups comprise 1 to 3 rings, either separated o condensed, containing approximately 6 to 18 ring carbon atoms; for instance, phenyl, naphthyl, diphenyl, indenyl, phenanthryl or anthranyl. Aryl radicals may optionally contain one or more substituents such as hydroxy, mercapto, halogen, alkyl, phenoxy, alkoxy, haloalkyl, nitro, cyano, dialkylamino, aminoalkyl, acyl, alkoxycarbonyl, etc.
"Alkenyl" refers to an alkyl radical possessing at least 2 C atoms and one or more unsaturated bonds.
"Cycloalkyl" refers to stable monocyclic or bicyclic radicals with 3 to 10-membered rings, saturated or partially saturated, and constituted only of carbon and hydrogen atoms, such as cyclohexyl or adamantyl. Unless otherwise stated in the detailed description, the term "cycloalkyl" also includes cycloalkyl radicals optionally substituted by one or more groups such as alkyl, halogen, hydroxy, amino, cyano, nitro, alkoxy, carboxy, alkoxycarbonyl, etc.
"Heterocycyl" refers to stable radicals with 3 to 15-membered rings, consisting of carbon atoms and up to five heteroatoms selected among nitrogen, oxygen and sulfur, preferably a 4- or 8-membered ring with one or more heteroatoms and, more preferably, a 5 or 6-membered ring with one or more heteroatoms. It may be aromatic or not. For the object of the invention, the heterocycle may contain a ring system, either monocyclic, bicyclic or tricyclic, optionally including condensed ring systems; optionally containing oxidized nitrogen, carbon or sulfur atoms; the nitrogen atom may be optionally quaternary; and the heterocyclyl radical may be partially or totally saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran, coumarin, morpholine, pyrrole, pyrazole, oxazole, isoxazole, triazole, imidazole, etc.
"Alkoxyl" refers to a group of the formula -ORa, where Ra is an alkyl radical defined as above; for instance, metoxy, ethoxy, propoxy, etc.
"Amino" refers to a group of the formula -NH₂, -NHRa or NRaRb, optionally quaternized, where Ra and Rb are alkyl radicals as defined above and independently selected; for instance methylamino, ethylamino, methylpropylamino, etc.
"Halogen" or "halo" refers to bromine, chloride, iodine or fluorine.

Mentions of substituted groups in the compounds of the present invention refer to the specified residue, which may be substituted in one or more available positions by one or more suitable groups; for instance, halogen as fluorine, chlorine, bromine and iodine; cyano; hydroxy; nitro; azido; alkanoyl as a Cl-6 group such as acyl and related; carboxamido; alkyl groups including approximately 1 to 12 carbon atoms or, more preferably, 1-3 carbon atoms; alkenyl and alkynyl groups including one or more unsaturated bonds and approximately 2 to 12 carbon atoms or, more preferably 2-6 carbon atoms; alkoxyl groups with approximately 1 to 12 carbon atoms and, more preferably 1-6 carbon atoms; aryloxyl as phenoxy; alkylthio groups including residues with one or more thioether bonds and approximately 1 to 12 carbon atoms or, more preferably, 1-6 carbon atoms; alkylsulfinyl groups including residues with one or more sulfinyl bonds and approximately 1 to 12 carbon atoms or, more preferably 1-6 carbon atoms; alkylsulfonyl groups including residues with one or more sulfonyl bonds and approximately 1 to 12 carbon atoms or, more preferably 1-6 carbon atoms; aminoalkyl with one or more N atoms and approximately 1 to 12 carbon atoms or, more preferably 1-6 carbon atoms; carbocyclic aryl with 6 or more carbon atoms, particularly phenyl or naphthyl and aralkyl as benzyl. Unless otherwise stated, a substituted group may optionally contain additional substituents, independent from each other, in every substitutable position.

It is well known that introduction of α-amino-β-lactams α-alkylated according to the formula (II) in open peptides of the type A₁-(beta-Lactam)-A₂, where A₁ and A₂ represent two amino acids, promotes the stabilization of a type II or II' β-turn within the donor NH of residue A₂ and C=O acceptor of residue A₁, as described in Angew. Chem. Int. Ed. 38, 3056-3058 (1999), J. Org. Chem. 66, 6333-6338 (2001) or J. Am. Chem. Soc. 125, 16243-16260 (2003).

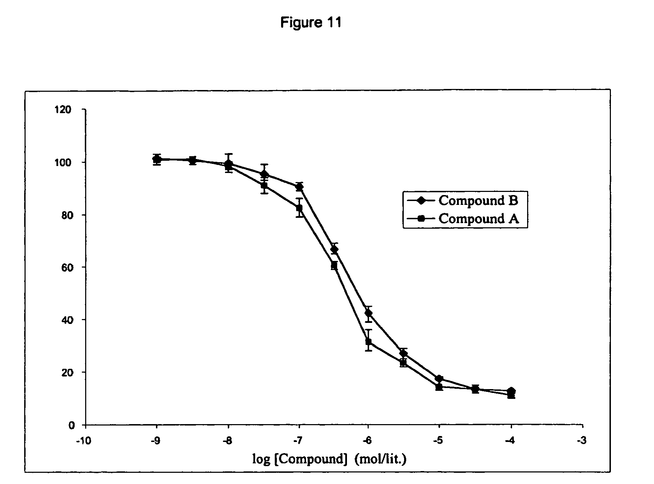

Surprisingly, we have found that introduction of α-amino-β-lactams in RGD cyclic pentapeptides forms a γ- turn at a position that depends on the (R) or (S) configuration of the Cα stereocenter of the azetidin-2-one ring contained in the (beta-Lactam) fragment, but is independent of substituents R², R³, R⁴ and R⁵. Accordingly, we have established that an (S) configuration at the Cα stereocenter of the β-lactam ring when R⁶ is H induces an inverse γ- turn within the donor N-H of the (beta-Lactam) fragment defined in the formula (II) and the acceptor C=O (Gly); conversely, an (R) configuration at the Cα when R⁶ is H induces a γ- turn with a hydrogen bond between N-H(Asp) and C=O(Arg). When R⁶ is different from H, a γ- turn with a hydrogen bond between N-H(Asp) and C=O(Arg) forms, irrespective of the (R) or (S) configuration at the β-lactam Cα carbon. The presence of at least one γ-turn has been determined in solution by NMR/Molecular Dynamics techniques (Examples 9-10). Therefore, whatever the case, the compounds of the invention always contain γ- turns and they have no tendency to form β- turns. And, more importantly, the compounds of the invention defined as above inhibit selectively and efficiently integrins of the type αᵥβ₃, α_{II}β₃, αᵥβ₅ and α₅β₁, irrespective of the kind of γ- turn (Example 11 and Figure 11).

In a variant of the invention, R¹ is not H. In this case, α-alkyl-α-amino-β-lactams of the formula (II) in RGD pseudopentapeptides of the formula (I) do not form β turns inside the cycle, as could be expect from prior art. In this variant R¹ is preferably an alkyl group, linear, branched or cyclic, containing 1 to 16 carbon atoms, preferably from 1 to 6 carbon atoms, or an alkylidenearomatic group defined by the formula -(CH₂)ₙAr where n is 1, 2, 3 or 4 and Ar is an aromatic or heteroaromatic group. The aromatic or heteroaromatic group is preferably phenyl, 1- or 2-naphthyl, 1-, 2- or 9-anthranyl, 2-, 3- or 4-diphenyl, 2-, 3-, or 4-pyridyl, 2- or 3-furyl, 2- or 3-thienyl, 1-, 2- or 3-pyrrolyl, 1-, 2-, 3-, 4- or 5-imidazolyl, 1-, 3-, 4- or 5-pyrazolyl, 2-, 4- or 5- oxazolyl, 3-, 4- or 5- isoxazolyl, 2-, 4- or 5-thiazolyl, 3-, 4- or 5- isothiazolyl. These groups may contain substituents (mono-, di- tri-, tetra- o penta- substituted) independently chosen from methyl, ethyl, n-propyl, iso-propyl, n-butyl, iso-butyl, sec-butyl, tert-butyl, CF₃, F, OMe, NMe₂ or NH₂. In a preferred variant R¹ is -CH₂Ph.

In another variant R¹ is an alkylpolyfluorinated group defined by the formula -(CH₂)ₘRf where m is 0,1, 2, 3 or 4 and Rf is CF₃, C₂F₅, n-C₃F₇, n-C₄F₉, n-C₅F₁₁, n-C₆F₁₃ or C₆F₅; alternatively, R¹ is a functionalized alkyl group defined by the formula -(CH₂)ₚX where p is 1, 2, 3 or 4 and X is OH, NH₂, SH or CO₂H.

In another variant of the invention R² is H or an alkyl group, linear or branched, containing from 1 to 15 carbon atoms; preferably H, CH₃, C₂H₅ or a functionalized C1-C4 alkyl group. In another variant R² is an alkylidenearomatic group defined by the formula -(CH₂)ₙAr, where n is 1, 2, 3 or 4 and Ar is an aromatic or heteroaromatic group.

In another variant of the invention R³ is selected among H, methyl, ethyl, allyl, benzyl or phenyl.

In another variant of the invention R⁴ is selected preferably among H, methyl, ethyl, allyl, iso-propyl, iso-butyl, benzyl, allyl, 4-hydroxybenzyl, hydroxymethyl, 1-hydroxyethyl, carboxymethyl, carboxyethyl, aminocarbonylmethyl, aminocarbonylethyl, 4-aminobutyl, 3-guanidylpropyl, thiomethyl, 2-(methylthio)ethyl or 3-indolylmethyl. Alternatively, it is an alkyl group, linear or branched, containing from 5 to 15 atoms or a functionalized Cl-C4 alkyl group.

In another variant of the invention R⁵ is selected preferably among H, methyl, ethyl, allyl, benzyl or phenyl.

In some compounds bridging of R⁴ and R⁵ is preferred to form a -(CH₂)ₙ- group, where n is 2, 3 or 4.

In another preferred variant R⁶ is H.

Alternatively, R¹ and R⁶ can also be bridged to form an -(CH₂)ₙ- cyclic group where n' is 3, 4 or 5.

Some typical compounds of the invention are collected in the examples, see for instance examples 7 and 8.

The invention includes cyclopeptides defined according to the formula (I), containing all the enantiomeric and diastereomeric forms of -(beta-Lactam)- fragments defined in the formula (II).

In formula (I) Arg represents the Arginine amino acid or a derivative thereof, Gly represents the Glycine amino acid or a derivative thereof, and Asp represents Aspartic acid or a derivative thereof. Configuration of the amino acids R and D in the sequence RGD is (L). These amino acids may contain protecting groups.

Derivatized compounds include prodrugs transformable *in vivo* into the compounds of the invention. Such derivatized compounds are evident for an expert in the art and include, depending on the functional groups presented in the molecule and without limitation, esters, amino acid esters, phosphate esters, sulfonate esters of metal salts, carbamates and amides.

Particularly preferred are the derivatives or prodrugs that increase the bioavailability of the compounds of the invention when such compounds are administered to patients (for example, allowing oral administration to get an easier absorption in blood) or improve the administration of the original compound for a biological behavior with respect to the original species (for instance, in brain or lymphatic system).

The invention also includes compounds protected at the amino group by protecting groups, defined as chemical fragments able to prevent (block) the modification of the amino group in most chemical reactions, but easily removable under certain conditions after a transformation in a different position of the molecule. Preferred protecting groups are BOC, ALLOC, FMOC, CBZ, methoxycarbonyl, ethoxycarbonyl, benzyl, PBF, acetyl or benzoyl.

Acronyms of the protecting groups mentioned above are defined as follows:
- ALLOC: -tert-Allyloxycarbonyl.
- Bn: -Benzyl
- BOC: -tert-Butoxycarbonyl.
- CBZ: -Benzyloxycarbonyl.
- FMOC: -9-Fluorenylmethoxycarbonyl.
- PBF: -2,2,4,6,7-Pentamethyldihydrobenzofuran-5-sulfonyl.

In addition, it is possible to use the functional groups of the side chains of some amino acid residues or derivatized amino acids to immobilize the cyclic peptides of the invention onto different materials, such as polymers or proteins, chromatographic stationary phases or fluorescent dyes. They can also be immobilized introducing functional groups in the substituents R¹, R² or R⁴. This permits the use of the compounds of the invention in systems for analysis or diagnostic, owing to their ability to bind integrins. It can also be applied to systems for detection and separation of integrins.

Unless otherwise stated, the invention also includes compounds containing one or several isotopically enriched atoms, and their use as markers for diagnostic. Compounds defined as above, excepting those replacing hydrogen by deuterium or tritium, are inside the scope of the invention; for instance, the compounds with carbon atoms enriched by ¹³C or ¹⁴C, or with nitrogen atoms enriched by ¹⁵N.

Compounds of the formula (I) defined as above can be obtained by available synthetic procedures. One aspect of the invention refers to a procedure for the preparation of compounds of the formula (I) (or their protected derivatives containing, for example, Arg and Asp protected residues) by cyclization of the following peptide segments protected at the Arg and Asp residues:

H-Arg-Gly-Asp-(beta-Lactam)-OH

H-Gly-Asp-(beta-Lactam)-Arg-OH

H-Asp-(beta-Lactam)Arg-Gly-OH

H-(beta-Lactam)Arg-Gly-Asp-OH

Such cyclizations can be conducted using known peptide synthesis reagents collected, for instance, in Houben Weyl Methods of Organic Chemistry, Vol. E 22 b, pp.461-542 (2003), and protecting groups can be cleaved in a later step of the synthesis applying methods known in the state of the art.

Preparation of (beta-Lactam) fragments, defined according to formula (II) is known (see, for instance Angew. Chem. Int. Ed. 38, 3056-3058 (1999), J. Org. Chem. 66, 6333-6338 (2001), or J. Am. Chem. Soc. 125, 16243-16260 (2003)), and its incorporation in the former segments can be achieved using the compounds of the formula:

X-(beta-Lactam)-Y

Where X and Y represent, in each case and independently,:
- X:: H, C₆H₅-SO₂-, 4-NO₂C₆H₄-SO₂-, 2-NO₂C₆H₄-SO₂-, 2,4-(NO₂)₂C₆H₃-SO₂-, PBF-, ALLOC-, CBZ-, Bn-, BOC-, FMOC-, CHO-, Me₃Si-, ^{t}BuMe₂Si-
- Y:: OH, OLi, ONa, OK, OMe, O^{t}Bu, OBn, OC₆F₅, SC₆H₅, F, Cl

Reaction products can be purified, if necessary, by conventional methods such as crystallization, chromatography or trituration. When the procedures described above to obtain the compounds of the invention afford stereoisomer mixtures, they can be used as such, or they can be separated by conventional methods like preparative chromatography. In case stereocenters are present, compounds can be prepared in racemic form; alternatively, individual enantiomers can be prepared by enantioselective synthesis or by resolution.

Pharmaceutically acceptable salts of the compounds of the invention are prepared from original compounds containing basic or acid residues by conventional chemical methods. For instance, such compounds can be prepared from the free basic or acid forms with stoichiometric amounts of the appropriate base or acid in water, in an organic solvent, or in their mixtures.

In general, nonaqueous media are preferred, particularly diethylether, ethyl acetate, ethanol, isopropanol or acetonitrile. Examples of salts formed by acid addition include salts of mineral acids such as hydrochloride, hydrobromide, hydroiodide, sulfate, nitrate, phosphate, and salts of organic acids, for example acetate, maleate, fumarate, citrate, oxalate, succinate, tartrate, malate, mandelate, methanesulfonate and p-toluenesulfonate. Examples of salts formed by base addition include salts of inorganic bases, such as sodium, potassium, calcium, ammonium, magnesium, aluminum, lithium, and organic bases, for example, ethylenediamine, ethanolamine, N,N-dialkylethanolamine, triethanolamine and the salts of basic amino acids.

The compounds of the invention may present crystalline forms as free compounds or as solvates, both being included in the scope of the invention. Solvation techniques are well known in the state of the art. Adequate solvates are the pharmaceutically acceptable ones. In a particular variation, solvate is hydrate.

A preferred pharmaceutically acceptable form is crystalline, incorporated as such in a pharmaceutical composition. In the case of salts and solvates, residual solvents and additional ionic compounds have to be nontoxic. The compounds of the invention may present different polymorphic forms, all of them included in the invention.

The present invention additionally provides pharmaceutical compositions comprising a compound of the invention, or its pharmaceutically acceptable salt, derivative, prodrug or stereoisomer combined to a carrier, adjuvant or pharmaceutically acceptable vehicle, for administration to patients.

Examples of pharmaceutical composition include any solid composition (tablets, pills, capsules, granules) or liquid (solutions, suspensions or emulsions) for oral, topic or parenteral administration.

Another aspect of the invention refers to a method to treat or prevent diseases mediated by the over-expression of integrins αᵥβ₃, α_{II}β₃, αᵥβ₅ and α₅β₁ in humans and animals. The antiangiogenic and integrin inhibition properties described above are useful for the treatment of cancer, more particularly for metastasis of cancerous solid tumors. They are also useful for the treatment of thrombosis, restenosis following percutaneous transluminal coronary angioplasty (PTCA), rheumatoid arthritis or osteoporosis.

The following examples are presented as an illustration and do not limit the extension of the invention.

### EXAMPLES

Examples 1-8 show the methodology for the preparation of the new compounds according to the invention. The preparation of physiologically compatible salts is also described. In examples 9 and 10 the conformation in aqueous solution of the compounds prepared in examples 7 and 8 is described. Example 11 shows how to evaluate the biological activity of the compounds of the invention.

Acronyms of reagents or techniques used are defined as follows:
- DAPI: 4,6-Diamidine-2-phenylindole
- DIPA: Diisopropylamine
- DIPEA: Diisopropyletylamine
- DMEM: Dulbecco modified Eagle medium
- DMF: N,N-Dimethylformamide
- EDCI: 1-[3-(Dimethylamino)propyl]-3-ethylcarbodiimide hydrochloride
- HATU: O-(7-Azabenzotriazol-1-yl)-N,N,N',N'-tetramethyluronium hexafluorophosphate
- HOAT: 1-Hydroxy-7-azabenzotriazole
- HOBT: 1-Hydroxybenzotriazole hydrate
- LDA: Lithium diisopropylamide
- LHMDS: Lithium bis(trimethylsilyl)amide
- MS (ESI): Mass spectrometry (Electrospray ionization)
- THF: Tetrahydrofuran
- TMEDA: N,N,N',N'-Tetramethylethylenediamine

### EXAMPLE 1:

### Preparation of 2-NO₂C₆H₄-SO₆-(beta-Lactam)-OH.

### (beta-Lactam): R¹= CH₂Ph; R²=R³=R⁴=R⁵=R⁶= H; (3S) configuration, according to formula (II).

A suspension of (*S*)-α-benzylserine methyl ester (4.0 g, 19 mmol), 2-nitrobenzensulfonyl chloride (8.5 g, 38 mmol) and KHCO₃ (9.5 g, 95 mmol) in acetonitrile (150 mL) was refluxed for 16 h. To the "in situ" formed (*S*)-2-benzyl-2-methoxycarbonyl-1-(2-nitrobenzensulfonyl)aziridine and excess KHCO₃ cooled to 20 °C, was added allylamine (2.13 mL, 28.5 mmol) and the mixture was stirred for 16 h at room temperature. To the resulting suspension was added a saturated aqueous solution of NaHCO₃ (100 mL) and the mixture was extracted with EtOAc (3 x 50 mL). Combined organic phases were dried (anhydrous MgSO₄) and evaporated. Purification by column chromatography on silica gel (eluent: Hexane/EtOAc 3/1) afforded methyl (*S*)-3-allylamino-2-benzyl-2-(2-nitrobenzenesulfonylamino)propanoate (6.09 g 74%). To a solution of this compound in anhydrous THF (30 mL) cooled to 0 °C, LHMDS (1M in THF) (35 mL, 35 mmol) was added drop-wise during 5 min. and the resulting mixture was stirred at room temperature for 6 h. Then, a saturated aqueous solution of NaHCO₃ (50 mL) was added and the mixture was extracted with CH₂Cl₂ (3 x 5 mL). The organic phase was dried (MgSO₄) and evaporated to yield pure (*S*)-3-benzyl-3-(2-nitrobenzenesulfonylamino)-1-allylazetidin-2-one (5.13 g, 94%), which was dissolved in acetonitrile (30 mL). Sodium periodate (10.65 g, 50 mmol) and a catalityc amount of hydrated ruthenium trichloride (50 mg) were added to the solution at 0 °C and the mixture was stirred during 3h at the same temperature. An aqueous solution of HCl (0.1 M) (50 mL) was added and the mixture was extracted with EtOAc (3 x 30 mL). Combined organic phases were dried (MgSO₄) and evaporated to afford the tittle product. Overall yield: 4.05 g (51 %). [α]²⁰_{D} = -2.5 (c = 0.075, MeOH); ¹H NMR(500MHz, CD₃OD, δ): 7.77-8.12 (m, 4H, arom.), 7.31-7.21 (m, 5H, arom), 3.67 (d, 1H, NCH₂, β-Lactam, J= 6.0 Hz), 3.59 (s, 2H, PhCH%CO₂H), 3.56 (d, 1H. NCH₂, β-Lactam, J= 6.1 Hz). 3.28 (dd, 2H, CH₂Ph).

### EXAMPLE 2:

### Preparation of 2-NO₂C₆H₄-SO₂-(beta-Lactam)-OH.

### (beta-Lactam): R¹= CH₂Ph; R²=R³=R⁴=R^{s}=R⁶= H; (3R) configuration, according to formula (II).

A suspension of (*R*)-α-benzylserine methyl ester (2.1 g, 10 mmol), 2-nitrobenzensulfonyl chloride (2.2 g, 38 mmol) and KHCO₃ (5.0 g, 50 mmol) in acetonitrile (80 mL) was refluxed over 16 h. To the "in situ" formed (*R*)-2-benzyl-2-methoxycarbonyl-1-(2-nitrobenzensulfonyl)aziridine and excess KHCO₃ cooled to 20 °C, was added 2-(*O-tert*-butyldimethylsililoxy)-ethylamine (1.75 g, 10 mmol) and the mixture was stirred for 20 h at room temperature. To the resulting suspension was added a saturated aqueous solution of NaHCO₃ (50 mL) and the mixture was extracted with EtOAc (3 x 25 mL). Combined organic phases were dried (MgSO₄) and evaporated under reduced pressure to afford the intermediate. The crude product was purified by column chromatography (silicagel-60, eluent EtOAc/Hx 1:4) yielding methyl (*R*)-3*-*(2*-tert-*butyldimethylsilyloxyethyl)-2-benzyl-2-(2-nitrobenzenesulfonyl amino)propanoate (3.75 g 68%). To a solution of this compound in anhydrous THF (30 mL), cooled at 0 °C, LHMDS (1 M in THF) (25 mL, 25 mmol) was added drop by drop in 5 min and the resulting mixture was stirred, at room temperature, for 6 h. Upon completion an aqueous solution of NaHCO₃ (sat.) (20 mL) was added and the mixture was extracted with CH₂Cl₂ (3 x 15 mL). The organic phase was decanted and dried (MgSO₄). The evaporation under reduced pressure to afford the pure (*S*)-3-benzyl-3-(2-nitrobenzensulfonylamino) -1-(2-*tert-*butyldimethylsilyloxyethyl)-azetidin-2-one (3.43 g, 97%). The crude was dissolved in acetone (50 mL). Over this solution, cooled at 0°C, were added, drop by drop, a water (9 mL) solution of chromium trioxide (3.3 g, 33 mmol), sulfuric acid (2.86 mL) and hydrofluoric acid (48%) (0.8 mL, 8 mmol). The resulting solution was stirred, at room temperature, for 1 h. Then, isopropanol (5 mL) and water (100 mL) were successively added. The aqueous phase was extracted with EtOAc (3 x 20 mL), dried (Na₂SO₄) and evaporated. The crude product was purified by column chromatography (silicagel-60, eluent hexanes/EtOAc 1:5). Overall yield: 2.43 g (58%). [α]²⁰_{D} = +2.8 (c = 0.10, MeOH).

### EXAMPLE 3:

### Preparation of 2-NO₂C₆H₄-SO₂-(beta-Lactam)-OH.

(*beta-Lactam*): R¹= CH₂Ph; R²=R³=R⁵=R⁶= H; R⁴= Ph; (3*S*), (1'*R*) configuration, according to formula (II).

The procedure of example 2 was followed, starting from (*S*)-α-benzylserine (2.1 g, 10 mmol), and (*R*)-2-(*O*-tert-butyldimethylsilyloxy)-2-phenyl-ethylamine (2.51 g, 10 mmol). Overall yield: 2.18 g (44%). mp = 195-7 °C; [α]²⁰_{D} = -42.8 (c = 0.75, CH₂Cl₂); ¹H NMR(500MHz, CO(CD₃)₂, δ): 7.21-7.89 (m, 14H, arom.), 5.45 (s, 1H, PhCHCO₂H), 3.79 (d, 1H, NCH_{2.} β-Lactam, J= 6.0 Hz), 3.42 (d, 1H, NCH_{2,} β-Lactam, J= 5.6 Hz), 3.32 (broad s, 2H, CH₂Ph).

### EXAMPLE 4:

### Preparation of 2-NO₂C₆H₄-SO₂-(beta-Lactam)-OH.

(*beta-Lactam*): R¹= CH₂Ph; R²=R³=R⁵=R⁶= H; R⁴= Ph; (3*S*), (1'*S*) configuration, according to formula (II).

The procedure of example 2 was followed, starting from (*S*)-α-benzylserine (2.1 g, 10 mmol), and (*S*)-2-(*O*-tert-butyldimethylsilyloxy)-2-phenyl-ethylamine (2.51 g, 10 mmol). Overall yield: 2.58 g (52%). [α]²⁰_{D} = + 2.4 (c = 0.05, MeOH); ¹H NMR(500MHz, CD₃OD, δ): 7.77-8.30 (m, 4H, arom.), 6.74-7.2 (m, 5H, arom), 5.07 (s, 1H, PhCHCO₂H), 3.70 (d, 1H, NCH₂ β-Lactam, J= 6.3 Hz), 3.13 (dd, 2H, CH₂Ph), 3.11 (d,1H, NCH₂ β-Lactam, J= 6.6 Hz),

### EXAMPLE 5:

### Preparation of H-(beta-Lactam)-OMe.

(*beta-Lactam*): R¹= CH₂(2-Naphthyl); R²= CH₂CH(CH₃)₂; R³=R⁴=R⁵=R⁶= H; (3R, 4R) configuration, according to formula (II).
**a) (3*R*,4*R*)-1-[Bis(trimethylsilyl)methy]-3-[(4*S*,5*R*)-4,5-diphenyl-2-oxo-oxazolidin-3-yl]-4-isobutyl-3[-(2-naphthyl)methyl]azetidin-2-one:** To a solution of dry DIPA (0.92 mL, 6.5 mmol) in THF (30 mL) cooled at -78 °C under CO₂/acetone bath, was added a solution of n-BuLi (2.5 M in hexane) (2.6 mL, 6.5 mmol) and the resulting LDA solution was stirred for 30 min at room temperature. A solution of (3*S*,4*R*)-1-[bis(trimethylsilyl)methyl]-3-[(4*S*,5*R*)-4,5-diphenyl-2-oxo-oxazolidin-3-yl]-4-isobutyl-azetidin-2-one (2.61 g, 5 mmol) [prepared according to description in Chem. Eur. J., 3, 1432-1441 (1997)] in THF (40 mL) was added over the previously formed LDA and the resulting mixture was stirred at -78 °C for 10 min. Then, a solution of 2-bromomethylnaphthalene (3.28 g, 15 mmol) in anhydrous THF (30 mL) was added and the mixture was stirred for 16 h while the temperature was slowly warmed to room temperature. The reaction mixture was poured with CH₂Cl₂ (200 mL) and was successively washed with saturated aqueous NH₄Cl (50 mL), HCl (1 M) (50 mL) and NaHCO₃ (sat.) (50 mL). The organic layer was dried (MgSO₄) and evaporated under reduced pressure to afford a crude, which was purified by column chromatography (silicagel-60, eluent EtOAc/Hex 1:6). Yield: 2.58 g (78%). m.p.: 187-188 (EtOH); [α]²⁰_{D} = - 0.012 (c = 0.5, EtOH); IR(cm⁻¹, KBr): 1760.7, 1741.7 (C=O), 845.3 (C-Si). ¹H NMR(500MHz, CDCl₃, δ): 8.00 (s, 1H, H-1 Naphth), 7.83 (d, 1H, J= 7.5 Hz, H-8 Naphth), 7.78 (d, 1H, J= 8.3 Hz, H-5, Naphth) 7.71 (d, 1H, J= 8.4 Hz , H-3 Naphth), 7.62 (d, 1H, J= 7.8 Hz, H-4 Naphth), 7.45 (m, 2H, H-6, H-7 Naphth), 7.01-6.99 (m, 10H, Ph), 5.60 (d, 1H, J= 7.2 Hz, OCHPh), 5.02 (d, 1H, J= 7.2 Hz, NCHPh), 3.95 (t, 1H, NCH/Bu), 3.29 (d, 1H, J= 15.8 Hz, CH₂Naphth), 3.09 (d, 1H, J= 15.8 Hz, CH₂Naphth), 2.02 (s, 1H, CHSiMe₃), 1.63 (m, 1H, CH₂CHMe₂), 1.26 (m, 2H, CH₂CHMe₂), 0.86 (m, 6H, CH₂CHMe₂), 0.28 (s, 9H, SiMe₃), 0.04 (s, 9H, SiMe₃)
**b) (3*R*,4*R)-*3-[(4*S*,5*R*)*-*4,5-Diphenyl-2-oxo-oxazolidin-3-yl]-4-isobutyl-1-[(methoxycarbonyl) methyl]-3[-(2-naphthyl)methyl]azetldln-2-one:** To a solution of (3*R*,4*R*)-1-[bis(trimethylsilyl) methyl]-3-[(4*S*,5*R*)-4,5-diphenyl-2-oxo-oxazolidin-3-yl]-4-isobutyl-3-[-(2-naphthyl)methyl]azetidin-2-one (2.58 g, 3.9 mmol) in acetonitrile (10 mL), cooled to 0 °C, was added Ce(NH₄)₂(NO₃)₄ (11.5 g, 21 mmol) in H₂O (20 mL). The mixture was stirred at 0 °C for 10 min and at room temperature for 2h. Then, water (100 mL) and EtOAc (3 x 30 mL) were added and the organic phase was washed with NaHCO₃ (sat.) (50 mL), dried (MgSO₄) and evaporated under reduced pressure to yield crude (3*R*,4*R*)-3-[(4*S*,5*R*)-4,5-diphenyl-2-oxo-oxazolidin-3-yl]-1-formyl-4-isobutyl-3-[(2-naphthyl)methyl] azetidin-2-one (100%). To this material dissolved in MeOH (50 mL) were added Na₂CO₃ (0.70 g, 0.6 mmol) and aqueous NaHCO₃ (sat) (20 mL), and the mixture was stirred at room temperature for 90 min. Solids were filtered off and washed with MeOH (20 mL). The combined methanolic phases were poured with H₂O (400 mL)/CH₂Cl₂ (10 mL) and extracted with CH₂Cl₂ (3 x 50 mL). The organic layer was dried (MgSO₄), evaporated and the resulting crude was purified by column chromatography (silicagel-60, eluent EtOAc/Hex 1:3). Yield: 1.41 g (63%). To a suspension of NaH (0.11 g, 3.8 mmol) in anhydrous THF (25 mL) cooled to 0 °C was added, successively, (3*R*,4*R*)-3-[(4*S,*5*R*)-4,5-diphenyl-2-oxo-oxazolidin-3-yl]-4-isobutyl-3[(2-naphthyl)methyl]azetidin-2-one (1.34 g, 3 mmol), methyl bromoacetate (0.39 mL, 4.2 mmol) and 15-crown-5 (0.13 g, 0.3 mmol) and the reaction mixture was stirred at room temperature for 2 h. Aqueous HCl (2 M) was added until strongly acidic pH and the mixture was extracted with CH₂Cl₂ (2 x 30 mL). The organic phases were dried (MgSO₄) and evaporated to afford a crude product, which was purified by column chromatography (silicagel-60, eluent EtOAc/Hx 1:1). Overall yield: 1.12 g (72%).
**c) (3*R*,4*R*)-3-Amino-4-isobutyl-1-[(methoxycarbonyl)methyl]-3-[(2-naphthyl)methyl]azetidin-2-one:** A suspension of (3*R*,4*R*)-3-[(4*S*,5*R*)-4,5-diphenyl-2-oxo-oxazolidin-3-yl]-4-isobutyl-1-[(methoxycarbonyl)methyl]-3-[(2-naphthyl)methyl]azetidin-2-one (1.04 g, 2 mmol) and Pd(OH)₂ (10% on carbon, 0.16 g) in methanol (20 mL) was stirred under hydrogen atmosphere (1 atm.) for 48 h. After this time the solids were filtered off over celite and the solvent was evaporated to give a mixture of 1,2-diphenylethane and the desired product, which was purified by column chromatography (silicagel-60, eluent EtOAc/hexanes 1:10, after MeOH/CH₂Cl₂ 1:5). Yield: 0.58 g (95%).

### EXAMPLE 6:

### Preparation of Boc-(beta-Lactam)-OH.

(*beta-Lactam*): R¹= CH₂Ph; R³=R⁴=R⁵=R⁶= H; R²= Me; (3*R*, 4*R*) configuration, according to formula (II).
**a) (3*R*,4*R*)-3-Benzyl-1-[bis(trimethylsilyl)methyl]-3-*tert*-butoxycarbonylamino-4-methyl-azetidin-2-one:** The procedure of the example 5-a) was followed, starting from (3*S*,4*R*)-1-[bis(trimethylsilyl)methyl]-3-[(4*S*,5*R*)-4,5-diphenyl-2-oxo-oxazolidin-3-yl]-4-methyl-azetidin-2-one (2.61 g, 5 mmol) and benzyl bromide (5.55 mL, 15 mmol), to afford (3,4*R*)-3-Benzyl-1-[bis(trimethylsilyl)methyl]-3-[(4*S*,5*R*)-4,5-diphenyl-2-oxo-oxazolidin-3-yl]-4-methyl-azetidin-2-one. Yield: 1.91 g (67 %). A suspension of this compound, di-*tert*-butyl dicarbonate (6.06 g, 26.4 mmol) and Pd(OH)₂ (10% on carbon, 0.24 g, catal.) in anhydrous ethanol (80 mL) was stirred under hydrogen atmosphere (160 psi) at 60 °C for 24 h. The solids were filtered off over celite, the filtrate was evaporated, and the resulting crude was purified by column chromatography (silicagel-60, eluent EtOAc/hexanes 1:30). Overall yield: 1.34 g (60%). m.p.: 66-69 °C; [α]²⁰_{D} = -10.5 (c = 1, CH₂Cl₂). ¹H NMR(500MHz, CDCl₃, δ): 7.29-7.18 (m, 5H, Ar), 4.98 (s, 1H, NH), 3.83-3.79 (m, 1H, CH₃CHN) 3.30 (d, 1H, J= 13.8 Hz, PhCHHC), 2.89 (d, 1H, J= 13.7 Hz, PhCHHC), 2.11 (s, 1H, CH(SiMe₃)₂), 1.52 (s, 9H, (CH₃)₃C), 1.39 (s, 3H, CH₃), 0.20 (s, 9H, SiMe₃), 0.13 (s, 9H, SiMe₃).
**b) (3*R*,4*R*)-3-Benzyl-3-*tert*-butoxycarbonylamino-1-(carboxymethyl)-4-methylazetidin-2-one:** A solution of (3*R*,4*R*)-3-benzyl-1-[bis(trimethylsilyl)methyl]-3-*tert-*butoxycarbonylamino-4-methyl-azetidin-2-one (1.34 g, 3 mmol) in dry acetonitrile (20 mL) was warmed up to 70°C and CsF (1.32 g, 9 mmol) was added. The reaction mixture was stirred at same temperature for 2 h, the solvent was evaporated and the resulting crude was dissolved in CH₂Cl₂ (30 mL). The organic layer was washed with water (20 mL), dried (MgSO₄) and evaporated to afford crude (3*R*,4*R*)-3-benzyl-1-(trimethylsilylmethyl)-3-*tert*-butoxycarbonylamino-4-methyl-azetidin-2-one, which was purified by column chromatography (silicagel-60, eluant EtOAc/Hx 1:2). Yield: 1.03 g (91%). TMEDA (1.02 mL, 6.8 mmol) and tBuLi (1.5 M in pentane) (4.5 mL, 6.8 mmol) were added drop-wise to a solution of the former β-lactam (1.03 g, 2.73 mmol) in THF (12 mL) cooled to 0°C under atmosphere of nitrogen. The mixture was stirred at the same temperature for 1 h and dry CO₂ was bubbled through the solution during 2 min. The mixture was quenched with NH₄Cl (2 mL) and was extracted with CH₂Cl₂ (3 x 5 mL). The organic layer was basified with NaOH (1 M), the aqueous solution was separated, acidified with HCl 6 M and extracted with EtOAc (3 x 10 mL) to obtain the acid compound. Yield: 0.47 g (45%). IR KBr cm⁻¹: 3500-2800 (OH, broad), 1768; 1753; 1714 (C=O); ¹H NMR(500MHz, CDCl₃, δ): 7.38-7.27 (m, 5H, Ar), 5.03 (s, 1H, NHBoc), 4.22 (d, 1H, J=15.1Hz, NCHHCO₂H), 4.18 (m, 1H, (CO)NCH), 3.95 (d, 1H, J=15.2Hz, NCHHCO₂H), 3.18 (s, 2H, CH₂Ph), 1.43 (s, 9H, CO₂CMe₃) 1.39 (d, 3H, CH₃, J=5.4Hz).

### EXAMPLE 7:

### Preparation of cyclo[Arg-Gly-Asp-(beta-Lactam)] trifluoroacetic salt.

(*beta-Lactam*): R¹= CH₂Ph; R²=R³=R⁴=R⁵=R⁸= H; configuration (3S), according to formula (II).
**a) 3-CBZ-Asp(O^{t}Bu)-(*beta-Lactam*)-OH:** To a solution of **(*S*)-3-amino-3-benzyl-1-**(methoxycarbonylmethyl)azetidin-2-one [H-(*beta-Lactam*)-OMe] (1.30 g, 3.02 mmol), and distilled N-methylmorpholine (0.83 mL, 7.55 mmol) in dry dichloromethane (20 mL) cooled to 0 °C, was added drop-wise a solution of the CBZ-Asp-(O^{t}Bu)-F acid fluoride (1.62 g, 4.99 mmol) in dry dichloromethane (20 mL). The reaction was stirred for 16h while warming slowly to room temperature. The reaction mixture was washed successively with an aqueous solution of HCl (0.1 M) (2 x 20 mL) and a saturated solution of NaHCO₃ (2 x 20 mL). The organic phase was dried (MgSO₄) and evaporated, and the resulting crude was purified by column chromatography (silicagel-60, eluant EtOAc/Hx 1:1). Yield (1.08 g, 65%). The resulting compound 3-CBZ-Asp(O^{t}Bu)-(*beta-Lactam*)-OMe (0.627 g, 1.13 mmol), was dissolved in THF (12 mL), a solution of LiOH.H₂O (71 mg, 1.7 mmol) in H₂O (6 mL) was added, and the resulting mixture was stirred at room temperature for 1 h. The mixture was acidified with 5% solution of citric acid, the THF was evaporated under reduced pressure and the aqueous phase was extracted with CH₂Cl₂ (3 x 5 mL). The combined organic phases were dried (MgSO₄) and the solvent was evaporated under reduced pressure to give the pure product (0.61 g, 100%). MS (ESI), m/z: 554.1. ¹HNMR (500MHz, DMSO_{d6}, δ): 7.37-7.26 (m, 10H, Ph), 6.96 (s, NH β-Lactam), 5.84 (d, 1H, J = 8.3 Hz, CbzNH), 5.12 (d, 1H, J = 12.2 Hz, OCH₂Ph), 5.08 (d, 1H, J = 12.2 Hz, OCH₂Ph), 4.49 (m, 1H, CHCH₂CO₂tBu), 4.12 (d, 1H, J = 17.6 Hz, CHCH₂CO₂H), 3.78 (d, 1H, J = 17.6 Hz, CHCH₂CO₂H), 3.67 (m, 1H, CH₂, β-Lactam), 3.62 (d, 1H, J = 4.9 Hz, CH₂, β-Lactam), 3.26 (d, 1H, 14.2 Hz, CH₂Ph), 3.20 (d, 1H, 12.7 Hz, CH₂Ph), 2.86 (dd, 1H, J = 17.1, 4.4 Hz, CH₂CO₂tBu), 2.58 (m, 1H, J = 17.1, 16.6, 6.3, 5.9 Hz, CH₂CO₂tBu), 1.41 (s, 9H, tBu).
**b) CBZ-Asp(O^{t}Bu)-(*beta*-*L*a*ctam*)-Arg(PBF)-Gly-OBn:** A solution of 3-CBZ-Asp(O^{t}Bu)-(*beta-Lactam*)-OH (0.291 g, 0.54 mmol) and H-Arg(PBF)-Gly-OBn (0.293g, 0.52 mmol) in CH₂Cl₂ (20 mL) was prepared under nitrogen atmosphere in a dried flask. To this mixture were added, consecutively, triethylamine (0.145 mL, 1.04 mmol), EDCI (0.16 g, 0.835 mmol) and HOBT (0.99 g, 0.73 mmol). The resulting mixture was stirred at room temperature for 20 h and then washed successively with aqueous solution of HCl (0.1 M) (2 x 10 mL) and a aqueous saturated solution of NaHCO₃ (2 x 10 mL). The organic phase was dried (MgSO₄) and evaporated under reduced pressure to yield a crude product, which was purified by column chromatography (silicagel-60, eluent MeOH/CH₂Cl₂ 1:25). Yield: 0.569 g (82%). MS (ESI), m/z: 1095.3; IR KBr, cm⁻¹: 3456, 3343, 1757, 1663, 1550, 1461. ¹H NMR(500MHz, DMSO_{d6}, δ): 8.38 (t, 1H, J= 5.6 Hz, NH-Gly), 8.23 (s, 1H, NH-β-Lactam), 8.50 (d , 1H, J= 8.3 Hz, NH-Asp), 8.12 (d, 1H, J= 7.8 Hz, NH-Arg), 7.35-7.22 (m, 15H, arom.), 5.12 (s, 2H, O CH₂Ph), 5.06 (d, 1H, J= 12.0 Hz, OCH₂Ph), 4.98 (d, 1H, J= 12.0 Hz, OCH₂Ph), 4.41 (m, 1H, Hα-Asp), 4.25 (m, 1H, Hα-Arg), 3.92 (dd, 1H, J= 5.9, 17.6 Hz, Hα-Gly), 3.85 (dd, 1H, J= 5.9, 17.6 Hz, Hα-Gly), 3.77 (d, 1H, J= 17.3Hz, Hα (β-Lactam), 3.70 (d, 1H, J= 17.3 Hz, Hα β-Lactam), 3.50 (d, 1H, J= 5.4 Hz, Hββ-Lactam), 3.36 (d, 1H, J= 5.4 Hz, Hβ,β-Lactam), 3.20 (d, 1H, J= 14.2 Hz, CH₂Ph β-Lactam), 3.09 (d, 1H, J= 14.2 Hz, CH₂Ph β-Lactam), 3.00 (m, 2H, Hδ-Arg), 2.92 (s, 2H, CH₂-Pbf), 2.53 (dd, 1H, J= 4.9, 16.1Hz, Hβ-Asp), 2.48 (s, 3H, Me-Pbf), 2.42 (s, 3H, Me-Pbf), 2.39 (dd, 1H, J= 9.3, 16.1Hz, Hβ-Asp), 2.48 (s, 3H, Me-Pbf), 2.42 (s, 3H, Me-Pbf), 2.39 (dd, 1H, J= 9.3, 16.1Hz, Hβ-Asp), 1.99 (s, 3H, Me-Pbf), 1.66 (m, 2H, Hβ-Arg), 1.49 (m, 2H, Hγ-Arg), 1.40 (s, 6H, 2xMe-Pbf), 1.34 (s, 9H, tBu).
**c) *cyclo*[Arg(PBF)-Gly-Asp(O^{t}Bu)-(*beta-Lactam*)]:** A suspension of CBZ-Asp(O^{t}Bu)-(*beta-Lactam*)*-*Arg(PBF)-Gly-OBn (86 mg, 0.08 mmol) and 10% Pd/C (9 mg) in ethanol (5 mL) was stirred under hydrogen atmosphere (1 atm.) for 16 h. The reaction mixture was filtered through a celite pad and the solvent was evaporated under reduced pressure. The crude (70 mg, 0.073 mmol, yield 92 %) was dissolved in dry DMF (20 mL), the resulting solution was cooled to 0°C under nitrogen atmosphere, and HATU (44 mg, 0.073 mol), HOAT (14 mg, 0.102 mmol) and DIPEA (0.076 mL, 0.44 mmol) were added consecutively. The reaction mixture was stirred for 30 min. at 0 °C checking the pH hourly with pH paper strips and keeping it adjusted to pH= 7-8. After 6 h additional DIPEA (0.013 mL, 0.073 mmol) was added to increase the pH value to 8-9. The mixture was stirred for 20 h at room temperature, the solvent was evaporated under reduced pressure at a temperature below 50°C, and the crude was dissolved in EtOAc (5 mL) and was washed with an aqueous solution of NaHCO₃ (5%). The crude product obtained after evaporation of solvent under reduced pressure was purified by preparative thin layer chromatography (eluent MeOH/CH₂Cl₂ 1:10). Yield: 17 mg, (28%). MS (MALDI-TOF), m/z: 853.02; IR KBr, cm⁻¹: 3446, 3386, 1752, 1730, 1663, 1550, 1259, 1160, 1130. ¹H NMR(500MHz, DMSO_{d6}, δ): 8.62 (t, 1H, J= 5.4 Hz, NH-Gly), 8.35 (s, 1H, NH-(β-Lactam), 8.26 (d , 1H, J= 7.8 Hz, NH-Asp), 8.11 (d, 1H, J= 8.8 Hz, NH-Arg), 7.28-7.11 (m, 5H, arom.), 4.50 (m, 1H, Hα-Asp), 4.17 (m, 1H, Hα-Arg), 3.93 (dd, 1H, J= 6.3,13.7 Hz, Hα-Gly), 3.75 (1H, d, J= 14.2 Hz, Hα β-Lactam), 3.50 (s, 1H, Hβ β-Lactam), 3.50-3.11 (m, 5H, Hα-Gly, Hα β-Lactam, Hβ β-Lactam, CH₂Ph), 3.00 (m, 2H, Hδ-Arg), 2.85 (s, 2H, CH₂-Pbf), 2.70 (dd, 1H, J= 9.3, 16.1Hz, Hβ-Asp), 2.45 (m, 3H, Me-Pbf), 2.43 (m, 1H, Hβ-Asp), 2.40 (s, 3H, Me-Pbf), 1.54 (m, 1H, Hβ-Arg), 1.46 (m, 3H, Hβ,Hγ-Arg), 1.40 (s, 6H, 2xMe-Pbf), 1.38 (s, 9H, tBu).
**d) *cyclo*[Arg-Gly-Asp-(*beta-Lactam*)] trifluoroacetate salt:** A solution of *cyclo*[Arg(PBF)-Gly-Asp(O^{t}Bu)-(*beta-Lactam*)] (18 mg, 0.02 mmol) in trifluoroacetic acid (0.3 mL) was stirred at 35 °C for 1 h. The solution was poured with isopropyl ether (10 mL) and the resulting precipitate was centrifuged off. The solid was washed with isopropyl ether (2 x 5mL) and dried at reduced pressure. Yield: 14 mg (100%). MS (MALDI-TOF), m/z: 658.58. ¹H NMR (500MHz, H₂O/D₂O 90/10, δ): 8.83 (s, 1H, NH-Arg), 8.62 (t, 1H, NH-Gly), 7.85 (d, 1H, J= 5.4 Hz, NH-Asp), 7.73 (s, 1H, NH-β-Lactam), 7.34-7.02 (m, 5H, arom.), 7.15 (s, 1H, NHω-Arg), 6.61 (s, 1H, NHω-Arg), 4.55 (m, 1H, Hα-Asp), 4.23 (dd, 1H, J= 7.23, 17.3 Hz, Hα-Gly), 3.94 (m, 1H, Hα-Arg), 3.92 (d, 1H, J=14.0 Hz, Hα β-Lactam), 3.83 (s, 1H, Hβ β-Lactam), 3.76 (d, 1H, J= 14.0 Hz, Hα β-Lactam), 3.63 (s, 1H, Hβ β-Lactam), 3.50 (dd, 1H, J= 7.2, 17.3 Hz, Hα-Gly), 3.47 (d, 1H, J=14.1 Hz Hδ, β-Lactam), 3.12 (m, 2H, Hδ-Arg), 3.10 (d, 1H, J= 14.1 Hz, Hδ, β-Lactam), 2.85 (dd, 1H, J= 5.1, 17.5Hz, Hβ-Asp), 2.67 (dd, 1H, J= 8.0, 17.5 Hz, Hβ-Asp), 1.72 (m, 2H, Hγ-Arg), 1.62 (m, 1H, Hβ-Arg), 1.54 (m, 1H, Hβ-Arg).

### EXAMPLE 8:

### Preparation of cyclo[Arg-Gly-Asp-(beta-Lactam)] trifluoroacetate salt

(*beta-Lactam*): R¹= CH₂Ph; R²=R³=R⁴=R⁵=R⁶= H; configuration (3R), according to formula (II).

The procedure of example 7 was followed, starting from (*R*)-3-amino-3-benzyl-1-methoxycarbonylmethylazetidin-2-one (4.30g, 10 mmol). Overall yield: 1.12 g (17%). m.p. = 180-220°C (dec.); [α]²⁰_{D} = - 0.3 (c = 0.28, 1 M HCl). ¹H NMR(500MHz, H₂O/D₂O 90/10, δ) 9.22 (d, 1H, J= 5.3 Hz, NH-Arg), 8.93 (s, 1H, NH-p-Lactam), 8.59 (t, 1H, J= 6.3 Hz, NH-Gly), 8.11 (d, 1H, J= 7.6 Hz, NH-Asp), 7.03-7.40 (m, 5H, arom.), 7.13 (m, 2H, NH-Arg), 6.58 (m, 1H, NH-Arg), 4.80 (m, 1H, Asp), 3.98 (dd, 1H, J= 6.8Hz, CH₂, Hα-Gly), 3,86 (d, 1H, J= 15.7Hz, CH₂), 3.80 (m, 1H, CH₂), 3.20 (d, 1H, J=14.7Hz, CH), 3.19 (d, 1H, J= 13.9Hz, CH₂), 3,14 (dd, 2H, J= 7.0Hz, J= 13.5Hz, CH₂), 3.01 (d, 1H, J= 13.8Hz, CH₂), 2.77 (dq, 2H, J= 6.9Hz, J= 16.6Hz, CH₂, Hα-Asp), 1.78 (m, 2H, CH₂, Hβ-Arg), 1.55 (m, 2H, CH₂, Hγ-Arg).

### EXAMPLE 9:

### Conformation of cyclo[Arg-Gly-Asp-(beta-Lactam)] in aqueous solution, incorporating (beta-Lactam) of configuration (S), according to formula (II), where R¹= CH₂Ph; R²=R³=R⁴=R⁵=R⁶= H (Compound A).

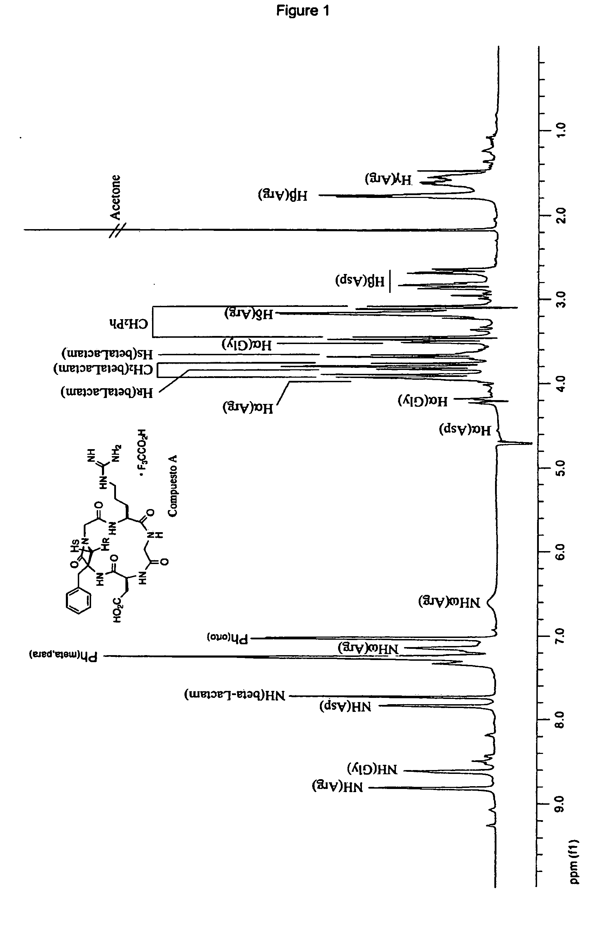

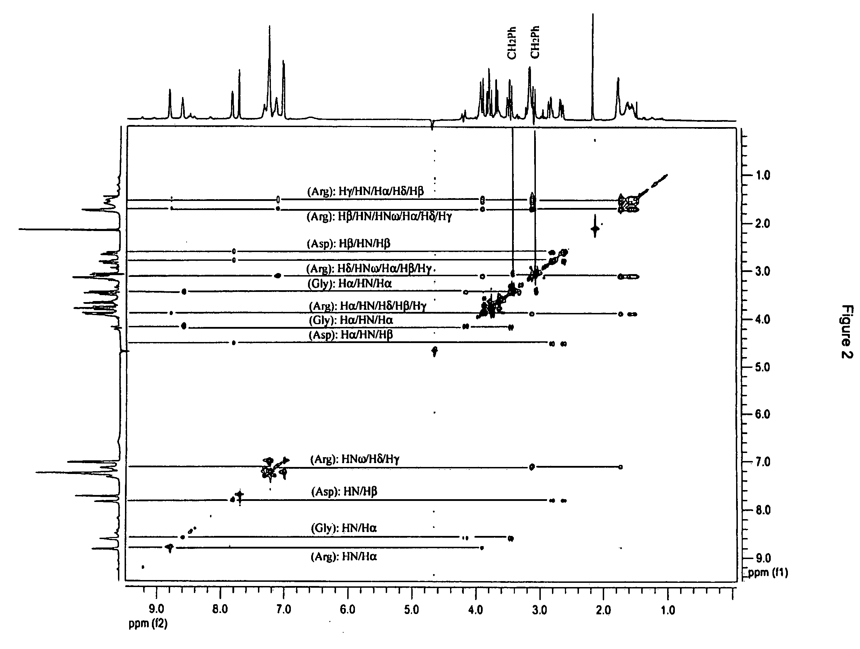

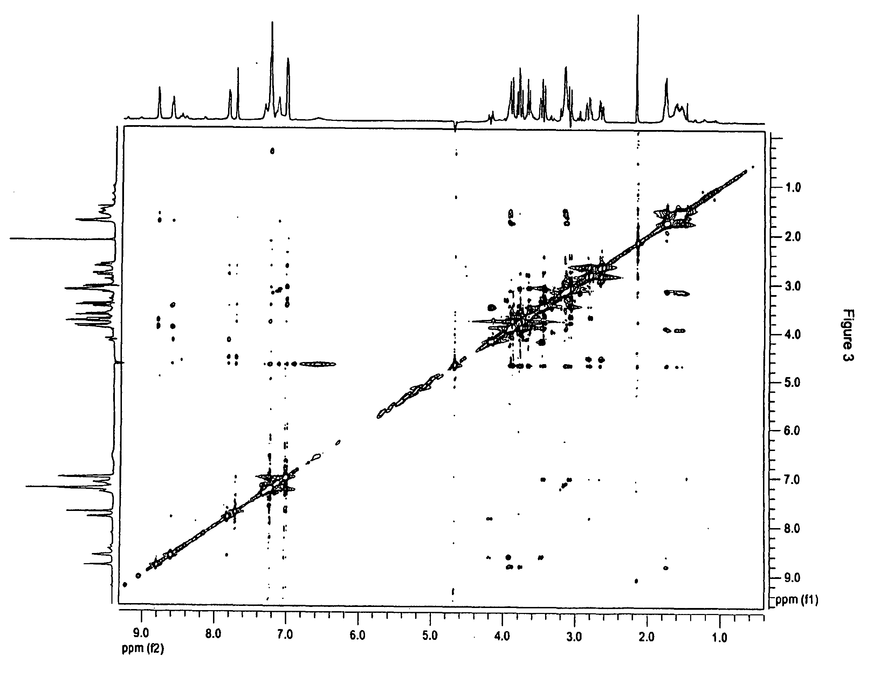

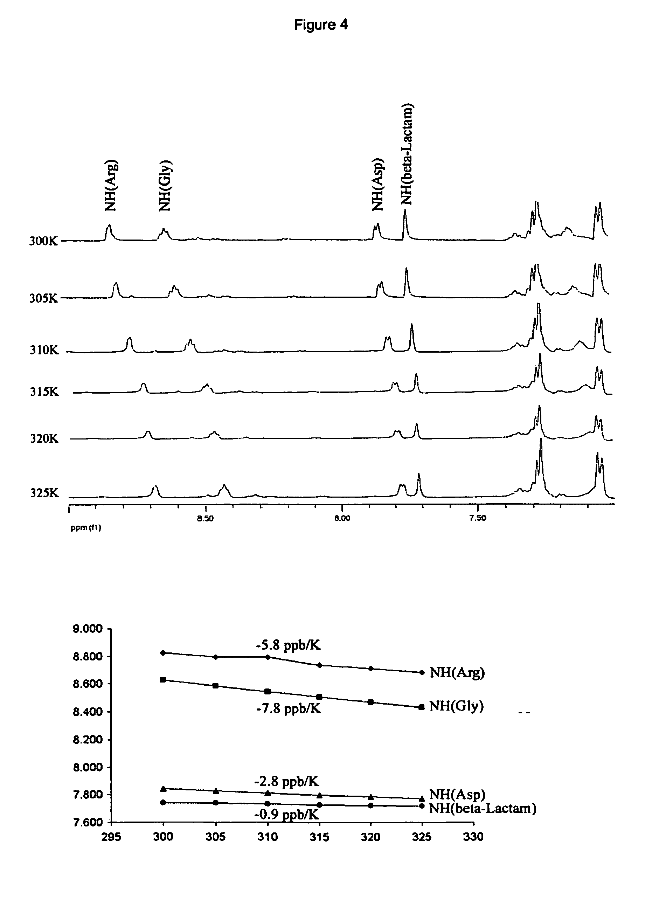

Compound A, prepared according to example 7, was dissolved at 10 mM concentration in distilled water containing 10% D₂O. The following experiments were conducted at 300K in a Bruker Avance-500 spectrometer equipped with a BBI probe with gradients in z axis: a) a ¹H monodimensional spectrum using "water suppression by excitation sculpting" pulse [J. Mag. Res., series A, 112, 275-279 (1995)] (Figure 1), b) a bidimensional spectrum TOCSY-WATERGATE "total correlation spectroscopy" [J. Mag. Res., 130, 162-168 (1998)] (Figure 2), c) a bidimensional spectrum ROESY-WATERGATE "rotating frame Overhauser enhancement spectroscopy" [J. Mag. Res., 63, 207-213 (1985)] with a mixing time of 200ms (Figure 3). Proton assignation in ¹H spectrum was based in TOCSY experiment correlations, using a sequential assignation procedure. In addition, inter-proton distances were measured from cross-peak integration in the ROESY spectrum. Chemical shift variation of amide protons with temperature was determined in the same solvent between 300K and 325K at 5K intervals (Figure 4).

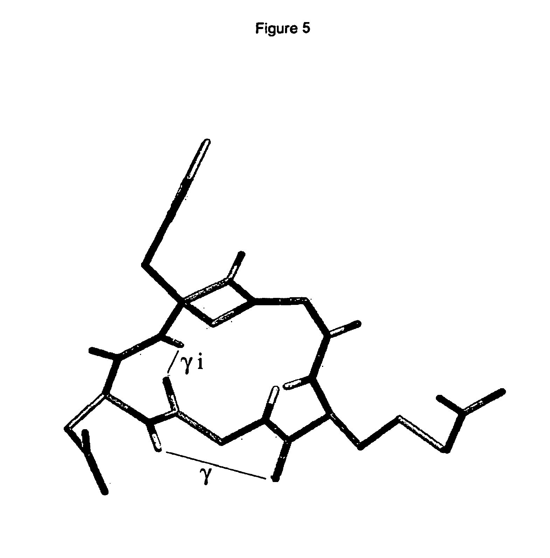

Taking as stable hydrogen bonds those showing thermal coefficients below -5 ppb/K, only NH(beta-Lactam) with -0.97 ppb/K and NH(Asp) with -2.8 ppb/K can be considered to participate in intramolecular hydrogen bonding. The high value observed for NH(Arg) (-5.59 ppb/K) confirmed the absence of any β turn between NH(Arg) and C=O(Asp). The three-dimensional structure of compound A (Figure 5) was determined in a water box at 300K from conformational group analysis by Molecular Dynamics incorporating restricted inter-proton distances, according to the method described in J. Am. Chem. Soc. 123, 2393-2404 (2001), and using the X-Plor (v2.9.2) program [J. Mag. Res., 160, 66-74 (2003)]. Compound A shows a reverse γ turn hydrogen-bonded between NH(beta-Lactam) and C=O (Gly), and a γ turn between NH(Asp) and C=O(Arg).

### EXAMPLE 10:

### Conformation of cyclo[Arg-Gly-Asp-(beta-Lactam)] In aqueous solution, incorporating (beta-Lactam) of configuration (R), according to formula (II), where R¹= CH₂Ph; R=R³=R⁴=R⁵=R⁶= H (Compounds B).

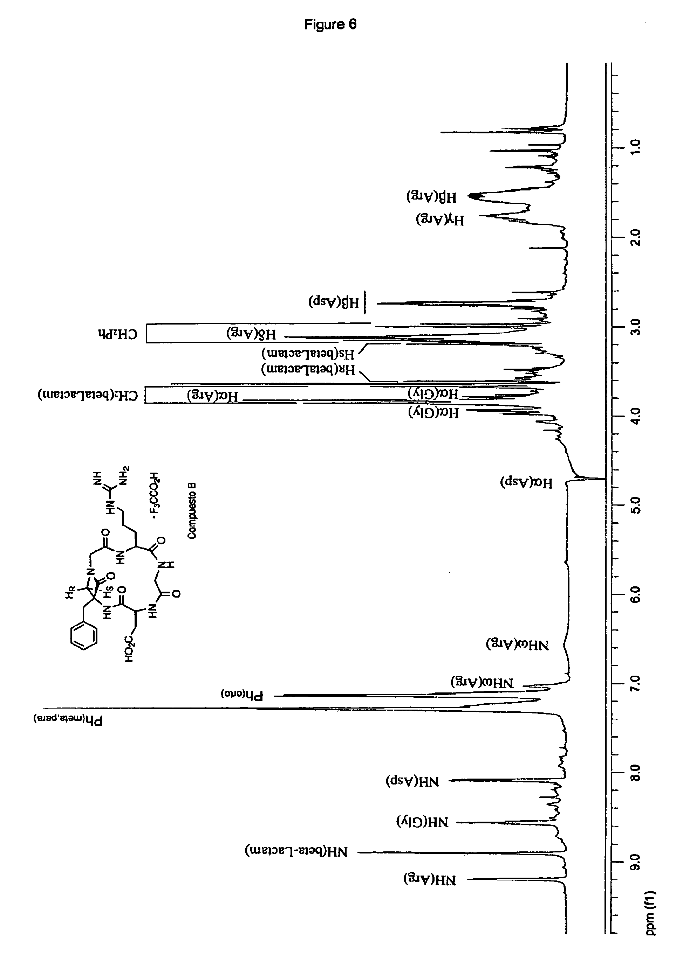

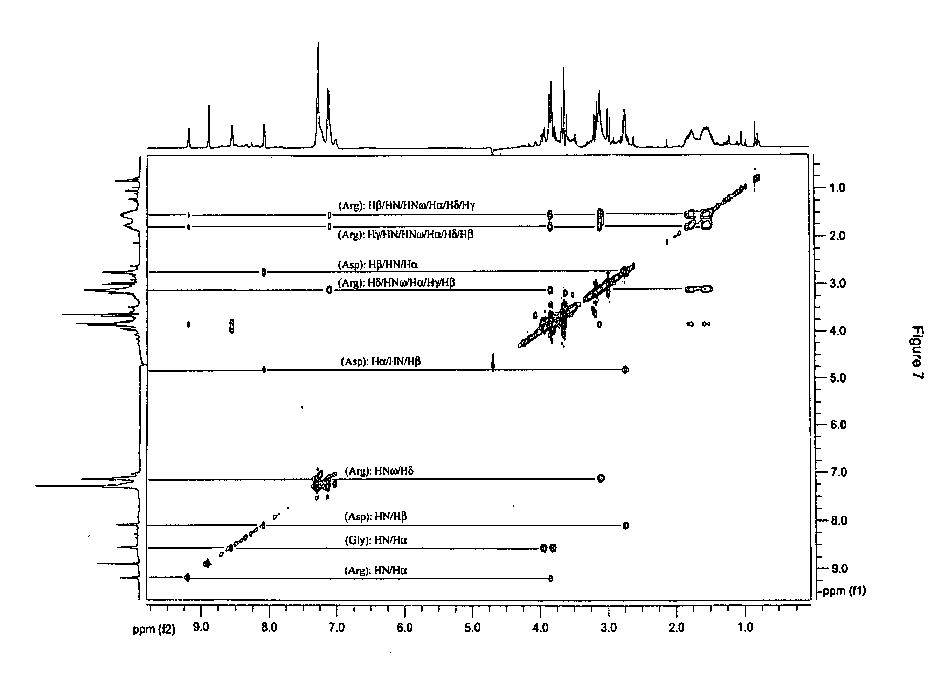

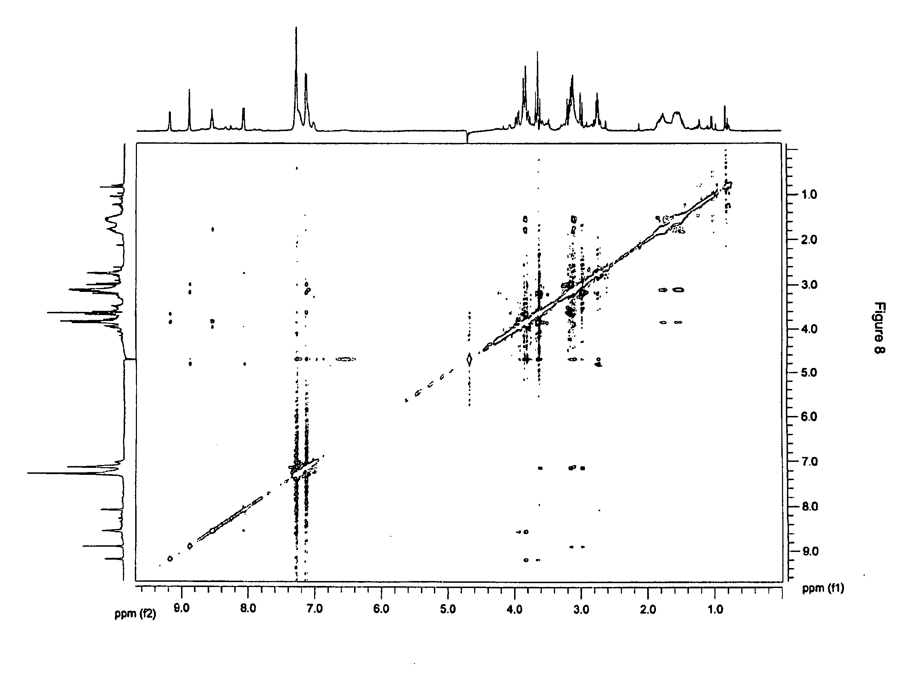

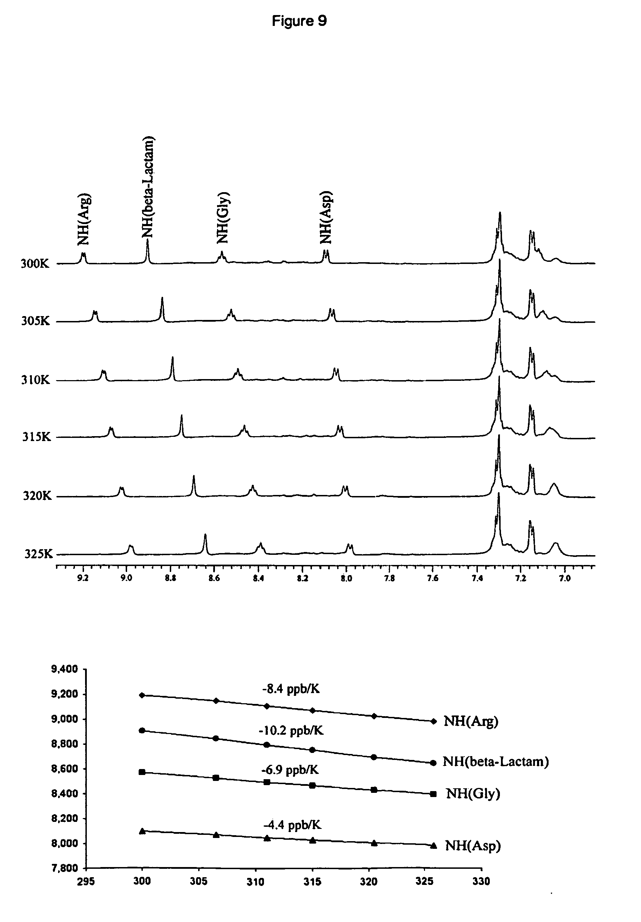

Compound B, prepared according to example 8, was dissolved at 5 mM concentration in distilled water containing 10% D₂O. Using the same techniques described for compound A, the following experiments were recorded: a) a ¹H spectrum using "water suppression by excitation sculpting" pulse (Figure 6), b) a bidimensional TOCSY-WATERGATE spectrum (Figure 7), c) a bidimensional ROESY-WATERGATE spectrum with a mixing time of 400ms (Figure 8). Proton assignation, inter-proton distance measurement and determination of the chemical shift variation of amide protons with temperature (Figure 9) were carried out under identical conditions to those described for compound A.

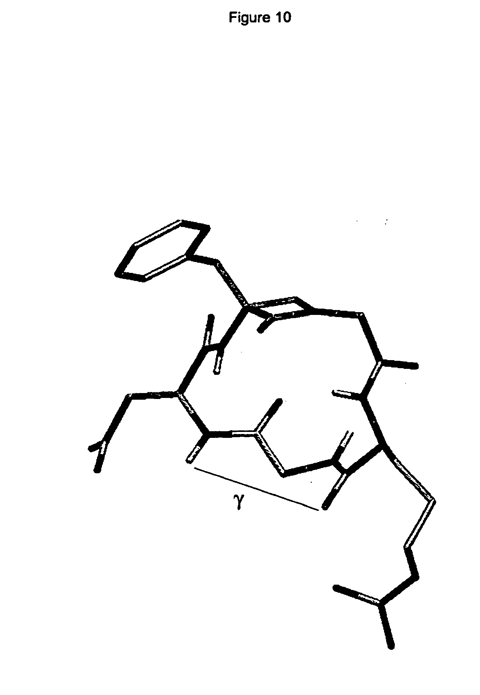

In compound B, only the NH(Asp) amide, with -4.4 ppb/K, forms an intramolecular hydrogen bond. The high value observed for NH(Arg) (-8.4 ppb/K) confirms the absence of any hydrogen-bonded β turn between NH(Arg) and C=O(Asp). The three-dimensional structure of compound B (Figure 10) was determined identically to compound A, showing a γ turn hydrogen-bonded between NH(Asp) and C=O (Arg).

### EXAMPLE 11:

### Endothelial cell adhesion assay of cyclo[Arg-Gly-Asp-(beta-Lactam)] containing (beta-Lactam) according to formula (II) of compounds A and B.

Human umbilical endothelial cells, HUVEC (BD Biosciences) were grown to 80% subconfluent state and then quickly harvested by 0.025% trypsin/EDTA (Sigma). Cells were preincubated with different concentrations of compounds compounds A, B, E, F and G for 20 min in CSC Medium (Sigma) prepared as described in examples 7 and 8.

The experiments were done in triplicate for each concentration and repeated three times in different experiments for a range of concentration between 10⁻⁴M and 10⁻⁹M at intervals of half range of magnitude. Peptide-treated cells (4x10⁴ cells/100µL/well) were plated onto a 96-well microplate which was precoated with vitronectin (10 mg/mL in PBS), and incubated for 1 h at 37°C, 5% CO₂/95% air to allow cell attachment. Cells were washed gently with PBS for three times to remove detached cells. Number of adherent cells was measured by DAPI fluorescent colorant method using cell flow cytometry (Becton Dinkinson Co).

Relative cell numbers are shown compared to the control experiment for each concentration with the error bars representing the standard error of the mean (SEM) (Figure 11). The figure clearly shows that compounds A, B compounds induce an inhibition of integrin αᵥβ₃-mediated adhesion at sub-micromolar concentration, probing that inhibition could be independent from the (*R*) or (*S*) configuration at the β-lactam a position, from the substitution at the α' position and from the type and position of the γ-turn.

## Claims

1. A compound of the formula (I):
*cyclo*[Arg-Gly-Asp-(beta-Lactam)] (I)
containing at least one γ- turn, where (beta-Lactam)- is a fragment of 3-amino-1-(2-carbonylmethyl)-azetidin-2-one of the formula (II) 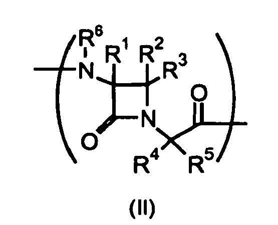 where R¹, R², R³, R⁴, R⁵ and R⁶ are selected independently from the group formed by hydrogen, unsubstituted or substituted alkyl, unsubstituted or substituted cycloalkyl, unsubstituted or substituted alkenyl, unsubstituted or substituted aryl, unsubstituted or substituted arylalkyl, unsubstituted or substituted heterocyclyl, unsubstituted or substituted heterocycloalkyl, -COR⁷, -C(O)OR⁷, -C(O)NR⁷R⁸-C=NR⁷, -CN, -OR⁷, -OC(O)R⁷, -S(O)ₜ-R⁷, - NR⁷R⁸, -NR⁷C(O)R⁸, -NO₂, -N=CR⁷R⁸or halogen;
where two of them can be bridged to form a cyclic substituent;
t is 1, 2 or 3,
R⁷ and R⁸ are selected independently from hydrogen, unsubstituted or substituted alkyl, unsubstituted or substituted cycloalkyl, unsubstituted or substituted alkenyl, unsubstituted or substituted aryl, unsubstituted or substituted heterocyclyl, unsubstituted or substituted alkoxyl, unsubstituted or substituted aryloxyl, or halogen;
their enantiomers, diastereomers or pharmaceutically acceptable salts.

2. A compound according to claim 1 **characterized by** an R¹ group selected among an alkyl group containing 1 to 6 carbon atoms or an alkylydenearomatic group defined by the formula - (CH₂)ₙAr where n is 1, 2, 3 or 4 and Ar is an aryl or heterocyclic aromatic group.

3. A compound according to claim 2 where R¹ is -CH₂Ph.

4. A compound according to any of the claims 1-3, **characterized by** an R⁴ group selected preferably among H, methyl, ethyl, allyl, iso-propyl, iso-butyl, benzyl, allyl, 4-hydroxybenzyl, hydroxymethyl, 1-hydroxyethyl, carboxymethyl, carboxyethyl, aminocarbonylmethyl, aminocarbonylethyl, 4-aminobutyl, 3-guanidylpropyl, thiomethyl, 2-(methylthio)ethyl, 3-indolylmethyl, preferably H.

5. A compound according to any of the claims 1-4, **characterized by** an R⁵ group selected among H, methyl, ethyl, allyl, benzyl or phenyl, preferably H.

6. A compound according to any of the claims 1-3, **characterized by** a bridged between R⁴ and R⁵ groups to form a -(CH₂)ₙ- cycle, where n is 2, 3 or 4.

7. A compound according to any of the claims 1-5, **characterized by** R², R³. R⁴, R⁵ and R⁶ where all of them are H.

8. A pharmaceutical composition comprising a compound defined according to any of the claims 1-7, their enantiomers, diastereomers or their mixtures.

9. A procedure for the obtention of a compound according to any of the claims 1-8, including:
a) the cyclization of the following pseudopeptides protected in the Arg and Asp residues:
H-Arg-Gly-Asp-(beta-Lactam)-OH
H-Gly-Asp-(beta-Lactam)-Arg-OH
H-Asp-(beta-Lactam)-Arg-Gly-OH
H-(beta-Lactam)-Arg-Gly-Asp-OH
where (beta-Lactam)- is a fragment of 3-amino-1-(2-carbonylmethyl)-azetidinone of the formula (II), as defined in claim 1;
b) optionally, the elimination of protecting groups.

10. A procedure according to claim 9 where the cyclization is made from a pseudopentapeptide of the formula H-Asp(OtBu)-(beta-Lactam)-Arg(PBF)-Gly-OH, its salts or activated esters.

11. A pseudopeptide of the formulae:
H-Arg-Gly-Asp-(beta-Lactam)-OH
H-Gly-Asp-(beta-Lactam)-Arg-OH
H-Asp-(beta-Lactam)-Arg-Gly-OH
H-(beta-Lactam)-Arg-Gly-Asp-OH
where (beta-Lactam)- is a fragment of 3-amino-1-(2-carbonylmethyl)-azetidinone of the formula (II) as defined in claim 1 and where Asp and Arg are optionally protected with protecting groups.

12. A pseudopeptide according to claim 11 of the formula H-Asp(OtBu)-(beta-Lactam)-Arg(PBF)-Gly-OH, their active salts or esters.

13. The use of a compound according to any of the claims 1-7 in drug manufacturing.

14. The use of a compound according to the claim 13 for the manufacture of a medicament for the treatment or prevention of diseases mediated by integrins of the type αᵥβ₃, α_{II}β₃, aᵥp₅ and α₅β₁ in humans and animals, and the disease is selecting from cancer, metastasis of cancerous solid tumors, thrombosis, restenosis following percutaneous transluminal coronary angioplasty (PTCA), rheumatoid arthritis or osteoporosis.

15. A system for integrin purification including a compound according to any of the claims 1-7.

16. Analysis or diagnostic systems including a compound according to any of the claims 1-7.

17. A material including a compound according to any of the claims 1-7 immobilized on a support.

18. A material according to claim 17 where the support is a polymer, a protein, a fluorescent dye or a chromatographic stationary phase.

## Patentansprüche

1. Verbindung mit der Formel (I):
Zyklo[Arg-Gly-Asp-(Beta-Lactam)] (I)
mit mindestens einer γ-Windung (Beta-Lactam) - einem Fragment von 3-Amino-1-(2-Carbonylmethyl)-Azetidin-2-Ona mit der Formel (II) 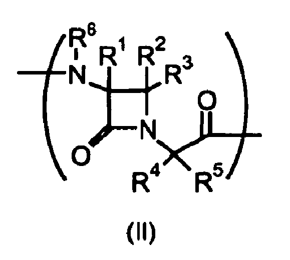 wobei R¹, R², R³, R⁴, R⁵ und R⁶ unabhängig aus der von Wasserstoff, substituiertem oder nicht substituiertem Alkyl, substituiertem oder nicht substituiertem Zykloalkyl, substituiertem oder nicht substituiertem Alkenyl, substituiertem oder nicht substituiertem Aryl, substituiertem oder nicht substituiertem Arylalkyl, substituiertem oder nicht substituiertem Heterozyklyl, substituiertem oder nicht substituiertem Heterozykloalkyl gebildeten Gruppe ausgewählt werden, -COR⁷, -C(O)OR⁷, - C(O)NR⁷R⁸-C=NR⁷, -CN, -OR⁷, -OC(O)R⁷, -S(O)ₜ-R⁷, -NR⁷R⁸, - NR⁷C(O)R⁸, -NO₂, -N=CR⁷R⁸ oder Halogen,
wobei zwei oder mehr dabon eine Brücke bilden können, damit ein zyklischer Substituent entsteht,
t 1, 2 oder 3 ist,
R⁷ und R⁸ unabhängig von Wasserstoff, substituiertem oder nicht substituiertem Alkyl, substituiertem oder nicht substituiertem Zykloalkyl, substituiertem oder nicht substituiertem Alkenyl, substituiertem oder nicht substituiertem Aryl, substituiertem oder nicht substituiertem Heterozyklyl, substituiertem oder nicht substituiertem Alkoxy, substituiertem oder nicht substituiertem Aryloxy oder Halogen ausgewählt werden,
seinen Enantiomeren, Diastereomeren oder pharmazeutisch akzeptablen Salzen.

2. Verbindung nach Formel 1, **dadurch gekennzeichnet, dass** die R¹ Gruppe aus einer Alkylgruppe ausgewählt wird, die 1 bis 6 Kohlenstoffatome aufweist, oder aus einer aromatischen Alkylidengruppe mit der Formel (CH₂)ₙAr, wobei n 1, 2, 3 oder 4 ist und Ar eine Arylgruppe oder eine aromatische Heterozyklylgruppe.

3. Verbindung nach Anspruch 2, bei der R¹ = -CH₂Ph.

4. Verbindung nach einem der Ansprücke 1 bis 3, **gekennzeichnet durch** eine R⁴ Gruppe, die bevorzugt unter H, Methyl, Ethyl, Alyl, Isopropyl, Isobutyl, Benzyl, 4-Hydroxybenzyl, Hydroxymethyl, 1-Hydroxyethyl, Carboxymethyl, Carboxyethyl, Aminocarbonylmethyl, Aminocarbonylethyl, 4-Aminobutyl, 3-Guanidilpropyl, Thiomethyl, 2-(Methylthio)ethyl, 3-Indolylmethyl, ausgewählt wird, bevorzugt H.

5. Verbindung nach einem der Ansprüche 1 bis 4, **gekennzeichnet durch** eine R⁵ Gruppe, die unter H, Methyl, Ethyl, Alyl, Benzyl oder Phenyl ausgewählt wird, bevorzugt H.

6. Verbindung nach einem der Ansprüche 1 bis 3, **gekennzeichnet durch** eine Brückenstruktur zwischen den Gruppen R⁴ und R⁵ zur Bildung eines Zyklus-(C_{H}2)ₙ-, bei dem n 2, 3 oder 4 ist.

7. Verbindung nach einem der Ansprüche 1 bis 5, **dadurch gekennzeichnet, dass** alle Gruppen R², R³, R⁴, R⁵ und R⁶ H sind.

8. Pharmazeutische Zusammensetzung bestehend aus einer nach irgendeinem der Ansprüche 1 bis 7 definierten Verbindung, ihren Enantiomeren, Diastereomeren oder Mischungen daraus.

9. Verfahren zum Erhalt einer Verbindung nach irgendeinem der Ansprüche 1 bis 8, bestehend aus:
a) Zyklisierung der folgenden in den Arg- und Asp-Resten geschützten Pseudopeptiden:
H-Arg-Gly-Asp-(Beta-Lactam)-OH
H-Gly-Asp-(Beta-Lactam)-Arg-OH
H-Asp-(Beta-Lactam)-Arg-Gly-OH
H-(Beta-Lactam)-Arg-Gly-Asp-OH
wobei (Beta-Lactam)- ein Fragment von 3-Amino-1-(2-Carbonylmethyl)-Azetidinon mit der Formel (II) nach Anspruch 1 ist;
b) wahlweise Entfernung der Schutzgruppen.

10. Verfahren nach Anspruch 9, bei dem die Zyklisierung ausgehend von einem Pseudo-Pentapeptid mit der Formel H-Asp(OtBu)-(Beta-Lactam)-Arg(PBF)-Gly-OH, seinen Salzen oder aktivierten Esthern erfolgt.

11. Pseudopeptide mit den Formeln:
H-Arg-Gly-Asp-(Beta-Lactam)-OH
H-Gly-Asp-(Beta-Lactam)-Arg-OH
H-Asp-(Beta-Lactam)-Arg-Gly-OH
H-(Beta-Lactam)-Arg-Gly-Asp-OH
wobei (Beta-Lactam) ein Fragment von 3-Amino-1-(2-Carbonylmethyl)-Azetidinon mit der Formel (II), wie unter Anspruch 1 definiert, ist und bei der Asp und Arg wahlweise durch Schutzgruppen geschützt sind.

12. Pseudopeptid nach Anspruch 11 mit der Formel H-Asp(OtBu)-(Beta-Lactam)-Arg(PBF)-Gly-OH, seine Salzen oder aktiven Esthern.

13. Verwendung der Verbindung nach einem der Ansprüche 1 bis 7 zur Herstellung von Arzneimitteln.

14. Verwendung einer Verbindung nach Anspruch 13 zur Herstellung eines Arzneimittels zur Behandlung von oder zur Vorbeugung gegen Krankheiten bei Mensch und Tier, bei denen Integrine des Typs ανβ₃, α_{∥}β₃, ανβ₅ und α₅β₁ beteiligt sind, wobei es sich bei der Krankheit um Krebs, Metastasen von soliden Krebstumoren, Thrombosen, Restenosen nach einer perkutanen transluminalen coronaren Angioplastie (PTCA) bzw. Herzkranzgefäß-Erweiterung, rheumatoiden Arthritis oder Osteoporose handeln kann.

15. System zur Reinigung der Integrine, die eine Verbindung nach einem der Ansprüche 1 bis 7 umfassen.

16. Analyse- oder Diagnosesysteme, die eine Verbindung nach einem der Ansprüche 1 bis 7 umfassen.

17. Material, das eine Verbindung nach einem der Ansprüche 1 bis 7 umfasst, welche auf einem Träger immobilisiert wird.

18. Material nach Anspruch 17, bei dem der Träger ein Polymer, ein Protein, ein fluoreszierender Farbstoff oder eine stationäre Chromatographiephase ist.

## Revendications

1. Composé de formule (I) :
cycle[Arg-Gly-Asp-(bêta-lactame)] (I)
contenant au moins une spire γ, (bêta-lactame)- étant un fragment de 3-amino-1-(2-carbonyleméthyle)- azétidine-2-one de formule (II) 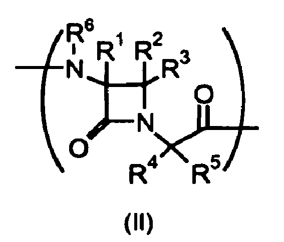 dans laquelle R¹, R² R³, R⁴, R⁵ et R⁶ sont indépendamment choisis parmi le groupe constitué d'hydrogène, d'alkyle substitué ou non substitué, de cycloalkyle substitué ou non substitué, d'alcényle substitué ou non substitué, d'aryle substitué ou non substitué, d'arylalkyle substitué ou non substitué, d'hétérocyclyle substitué ou non substitué, d'hétérocycloalkyle substitué ou non substitué, de -COR⁷, - C(O)OR, -C(O)NR⁷R^{B}-C=NR⁷, -CN, -OR⁷, -OC(O)R⁷, -S(O)ₜ-R⁷, - NR⁷R⁸, -NR⁷C(O)R⁸, -NO₂, -N=CR⁷R⁸ ou d'halogène ;
dans laquelle deux ou plusieurs d'entre eux peuvent former un pont pour constituer un substituant cyclique ;
t est 1, 2 ou 3,
R⁷ et R⁸ sont indépendamment choisis parmi l'hydrogène, l'alkyle substitué ou non substitué, le cycloalkyle substitué ou non substitué, l'alcényle substitué ou non substitué, l'aryle substitué ou non substitué, l'hétérocyclyle substitué ou non substitué, l'alcoxyle substitué ou non substitué, l'aryloxyle substitué ou non substitué, ou l'halogène ;
leurs énantiomères, diastéréomères ou sels pharmaceutiquement acceptables.

2. Composé, selon la formule 1, **caractérisé par** un groupe R¹ choisi parmi un groupe alkyle contenant de 1 à 6 atomes de carbone ou un groupe alkylidène aromatique défini par la formule (CH₂)ₙAr dans laquelle n est 1, 2, 3 ou 4 et Ar est un groupe aryle ou un groupe aromatique hétérocyclique.

3. Composé, selon la revendication 2, dans lequel R¹ est -CH₂Ph.

4. Composé, selon l'une quelconque des revendications 1 à 3, **caractérisé par** un groupe R⁴ de préférence choisi parmi H, le méthyle, l'éthyle, l'allyle, l'isopropyle, l'isobutyle, le benzyle, le 4-hydroxybenzyle, l'hydroxyméthyle, le 1-hydroxyéthyle, le carboxyméthyle, le carboxyéthyle, l'aminocarbonylméthyle, l'aminocarbonyléthyle, le 4-aminobutyle, le 3-guanidylpropyle, le thiométhyle, le 2-(méthylthio)éthyle, 3-indolylméthyle, de préférence H.

5. Composé, selon l'une quelconque des revendications 1 à 4, **caractérisé par** un groupe R⁵ choisi parmi H, le méthyle, l'éthyle, l'allyle, le benzyle ou le phényle, de préférence H.

6. Composé, selon l'une quelconque des revendications 1 à 3, **caractérisé par** une structure de pont entre les groupes R⁴ et R⁵ pour former un cycle-(CH₂)ₙ-, dans lequel n est 2, 3 ou 4.

7. Composé, selon l'une quelconque des revendications 1 à 5, **caractérisé en ce que** tous les groupes R², R³, R⁴, R⁵ et R⁶ sont H.

8. Composition pharmaceutique comprenant un composé défini selon l'une quelconque des revendications 1 à 7, ses énantiomères, diastéréomères ou ses mélanges.

9. Procédé pour l'obtention d'un composé, selon l'une quelconque des revendications 1 à 8, comprenant :
a) la cyclisation des pseudopeptides suivants protégés dans les résidus Arg et Asp :
H-Arg-Gly-Asp-(bêta-lactame)-OH
H-Gly-Asp-(bêta-lactame)-Arg-OH
H-Asp-(bêta-lactame)-Arg-Gly-OH
H-(bêta-lactame)-Arg-Gly-Asp-OH
dans lesquels (bêta-lactame)- est un fragment de 3-amino-1-(2-carbonyleméthyle)-azétidinone de formule (II), selon la revendication 1 ;
b) facultativement, l'élimination des groupes protecteurs.

10. Procédé, selon la revendication 9, dans lequel la cyclisation est réalisée à partir d'un pseudopentapeptide de formule H-Asp(OtBu)-(bêta-lactame)-Arg(PBF)-Gly-OH, ses sels ou esters activés.

11. Pseudopeptide de formules :
H-Arg-Gly-Asp-(bêta-lactame)-OH
H-Gly-Asp-(bêta-lactame)-Arg-OH
H-Asp-(bêta-lactame)-Arg-Gly-OH
H-(bêta-lactame)-Arg-Gly-Asp-OH
dans laquelle (bêta-lactame)- est un fragment de 3-amino-1-(2-carbonyleméthyle)-acétidinone de la formule (II), tel qu'il est défini dans la revendication 1, et dans laquelle Asp et Arg sont facultativement protégés avec des groupes protecteurs.

12. Pseudopeptide, selon la revendication 11, de formule H-Asp(OtBu)-(bêta-lactame)-Arg(PBF)-Gly-OH, ses sels ou esters actifs.

13. Utilisation du composé, selon l'une quelconque des revendications 1 à 7, dans la fabrication de médicaments.

14. Utilisation d'un composé, selon la revendication 13, pour la fabrication d'un médicament destiné au traitement ou à la prévention de maladies déclenchées par des intégrines du type ανβ₃, α_{∥}β₃, ανβ₃, et α₅β₃, chez les humains et les animaux, et la maladie est sélectionnée parmi le cancer, la métastase de tumeurs solides cancéreuses, la thrombose, la resténose faisant suite à une angioplastie coronaire transluminale percutanée (ACTP), l'arthrite rhumatoïde ou l'ostéoporose.

15. Système pour la purification d'intégrine comprenant un composé selon l'une quelconque des revendications 1 à 7.

16. Systèmes d'analyse ou de diagnostic comprenant un composé selon l'une quelconque des revendications 1 à 7.

17. Substance comprenant un composé selon l'une quelconque des revendications 1 à 7 immobilisé sur un support.

18. Substance, selon la revendication 17, dont le support est un polymère, une protéine, un colorant fluorescent ou une phase stationnaire chromatographique.
